# EUROPEAN PATENT APPLICATION

(11) **EP 0 970 946 A2**
(43) Date of publication of application: **12.01.2000**
(21) Application number: 99112722.6
(22) Date of filing: 01.07.1999
(51) Int. Cl.: C07C 235/76, C07C 235/20, C07C 233/05, C09J 139/00, H01L 21/58

(54) **Allylated amide compounds and die attach adhesives prepared therefrom**

(30) Priority: 02.07.1998 US 91509 P; 18.06.1999 US 336082
(71) Applicant: National Starch and Chemical Investment Holding Corporation, Wilmington, Delaware 19803-7663 (US)
(72) Inventor: Schultz, Rose Ann, Westford, Massachusetts 01886 (US); Herr, Donald, Flemington, New Jersey 08822 (US); Xiao, Chaodong, East Hanover, New Jersey 07936 (US)
(74) Representative: Held, Stephan, Dr.rer.nat., Dipl.-Chem.

(57) **Abstract**

Allylated amide compounds are formulated into curable compositions with a free radical curing agent, and optionally, one or more fillers, for use as adhesives in the manufacture of microelectronic devices.

## Description

The priority of provisional application 60/091,509, filed 02 July 1998, is claimed under 35 USC 119(e).

This invention relates to allylated amide compounds and adhesives prepared from those compounds.

Adhesive compositions, particularly conductive adhesives, are used for a variety of purposes in the fabrication and assembly of semiconductor packages and microelectronic devices. The more prominent uses are the bonding of integrated circuit chips to lead frames or other substrates, and the bonding of circuit packages or assemblies to printed wire boards.

The requirements for conductive adhesives in electronic packaging are that they have good mechanical strength, curing properties that do not affect the component or the carrier, and thixotropic properties compatible with existing application equipment currently used in the industry.

Another important aspect of an adhesive bonding or interconnection technology is the ability to rework the bond. For single chip packaging involving high volume commodity products, a failed chip can be discarded without significant loss. However, it becomes expensive to discard multi-chip packages with only one failed chip; consequently, the ability to rework the failed chip would be a manufacturing advantage. Today, one of the primary thrusts within the semiconductor industry is to develop adhesives that will meet all the requirements for adhesive strength and flexibility, but that will also be reworkable.

Conventional adhesive technology uses low viscosity thermosetting organic materials, the most widely used being epoxy systems. In order to achieve the required mechanical performance, relatively high molecular weight thermoplastics would be the preferred compositions for adhesive materials. These materials, however, have high viscosity or even solid film form, which are drawbacks to the manufacturing process. Therefore, there is a need for new adhesives that are easily dispensable to conform with automated manufacturing processes and that are reworkable.

This invention relates to allylated amide compounds that can be used in curable compositions. Allylated in this context means that allyl moieties are bonded to the nitrogen of amide moieties.

In another embodiment, this invention is an adhesive composition for use in electronic devices that comprises one or more allylated amide compounds, a curing initiator, and optionally, one or more fillers. The composition optionally may also contain mono- or polyfunctional vinyl compounds.

In another embodiment, this invention is the cured adhesive that results from the just described curable adhesive composition.

In another embodiment, this invention is a electronic assembly comprising an electronic component bonded to a substrate with a cured adhesive composition prepared from a composition comprising one or more allylated amide compounds, a curing initiator, optionally one or more fillers, and optionally one or more mono- or polyfunctional vinyl compounds.

In another embodiment, this invention is a method for adhering an electronic component to a substrate with a cured adhesive prepared from a composition comprising one or more allylated amide compounds, a curing initiator, optionally one or more fillers, and optionally, one or more mono- or polyfunctional vinyl compounds, the method comprising applying the adhesive to the component or the substrate, contacting the component and the substrate, and curing the adhesive in situ.

The allylated amide compounds, and vinyl compounds, used in the adhesive compositions of this invention are curable compounds, meaning that they are capable of polymerization, with or without crosslinking. As used in this specification, to cure will mean to polymerize, with or without crosslinking. Cross-linking, as is understood in the art, is the attachment of two polymer chains by bridges of an element, a molecular group, or a compound, and in general will take place upon heating. As cross-linking density is increased, the properties of a material can be changed from thermoplastic to thermosetting.

It is possible to prepare polymers of a wide range of cross-link density by the judicious choice and amount of mono- or polyfunctional compounds. The greater proportion of polyfunctional compounds reacted, the greater the cross-link density. In order to provide thermoplastic properties, adhesive compositions are prepared from mono-functional compounds to limit the cross-link density. However, a minor amount of poly-functional compounds can be added to provide some cross-linking and strength to the composition, provided the amount of poly-functional compounds is limited to an amount that does not diminish the desired thermoplastic properties. Within these parameters, the strength and elasticity of individual adhesives can be tailored to a particular end-use application.

The cross-link density can also be controlled to give a wide range of glass transition temperatures in the cured adhesive to withstand subsequent processing and operation temperatures.

In those cases where it is necessary to rework the assembly, the electronic component can be pried off the substrate, and any residue adhesive can be heated until it softens and is easily removed.

In the inventive adhesive compositions, the allylated amide compounds, and vinyl compounds if used in combination with the allylated amide compounds, will be present in the curable package adhesive compositions in an amount from 2 to 98 weight percent based on the organic components present (excluding any fillers).

The adhesive compositions will further comprise at least one free-radical initiator, which is defined to be a chemical species that decomposes to a molecular fragment having one or more unpaired electrons, highly reactive and usually short-lived, which is capable of initiating a chemical reaction by means of a chain mechanism. The free-radical initiator will be present in an amount of 0.1 to 10 percent, preferably 0.1 to 3.0 percent, by weight of the allylated amide compound, or combination of both allylated amide and vinyl compounds (excluding any filler). The free radical curing mechanism gives a fast cure and provides the composition with a long shelf life before cure. Preferred free-radical initiators include peroxides, such as butyl peroctoates and dicumyl peroxide, and azo compounds, such as 2,2'-azobis(2-methyl-propanenitrile) and 2,2'-azobis(2-methyl-butanenitrile).

Alternatively, the adhesive compositions may contain a photoinitiator, such as is sold by Ciba Specialty Chemicals under the trademark Irgacure, in lieu of the free-radical initiator, and the curing process may then be initiated by UV radiation. The photoinitiator will be present in an amount of 0.1 to 10 percent, preferably 0.1 to 3.0 percent, by weight of the allylated amide compound, or combination of both allylated amide and vinyl compounds (excluding any filler). In some cases, both photoinitiation and free-radical initiation may be desirable. For example, the curing process can be started by UV irradiation, and in a later processing step, curing can be completed by the application of heat to accomplish a free-radical cure.

In general, these compositions will cure within a temperature range of 50° to 250°C, and curing will be effected within a length of time of less than one minute to four hours. As will be understood, the time and temperature curing profile for each adhesive composition will vary, and different compositions can be designed to provide the curing profile that will be suited to the particular industrial manufacturing process.

Ease of application, even when thermoplastic properties are desired for the adhesive, is achieved by using relatively low molecular weight reactive oligomers or pre-polymers and curing these *in situ* after application to the electronic component or substrate. Applying the materials in an uncured state gives high processibility, and the resultant cured thermoplastic adhesive provides high mechanical performance.

Suitable conductive fillers for the adhesives are silver, copper, gold, palladium, platinum.

### Allylated Amide Compounds

The allylated amide compounds suitable for use in the compositions of this invention have a structure represented by the formulas A and B as depicted here:

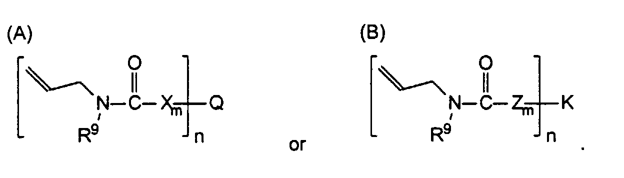

As used throughout this specification, the notation C(O) refers to a carbonyl group. For these specific formulae, when lower case "n" is the integer 1, the compound will be a mono-functional compound; and when lower case "n" is an integer 2 to 6, the compound will be a poly-functional compound.

Formula A represents those compounds in which:
R⁹ is H, an alkyl or alkyleneoxy group having 1 to 18 carbon atoms, allyl, aryl, or substituted aryl having the structure 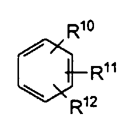 in which R¹⁰, R¹¹, and R¹² are independently H or an alkyl or alkyleneoxy group having 1 to 18 carbon atoms;
each X independently is an aromatic group selected from the aromatic groups having the structures (I) through (V): 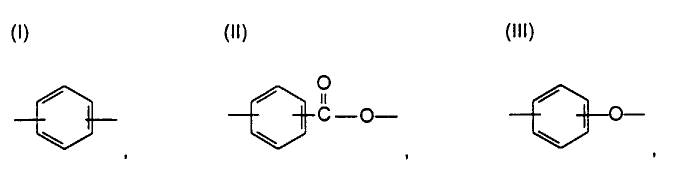 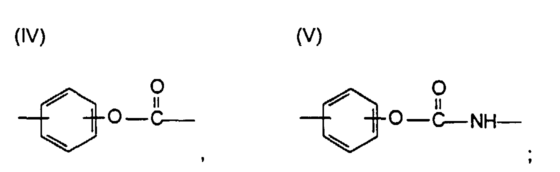
and Q is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteroatom present may or may not be directly attached to X;
or Q is a urethane having the structure: 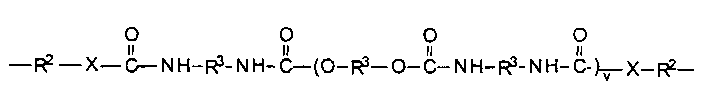 in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50;
or Q is an ester having the structure: 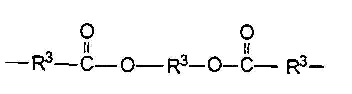 in which R³ is an alkyl or alkyloxy chain having up to 100 atoms in the chain, which chain may contain aryl substituents;
or Q is a siloxane having the structure:
   ―(CR¹₂)ₑ―[SiR⁴―O]_{f}―SiR⁴₂―(CR¹₂)_{g}― in which the R¹ substituent independently for each position is H or an alkyl group having 1 to 5 carbon atoms and the R⁴ substituent independently for each position is an alkyl group having 1 to 5 carbon atoms or an aryl group, and e and g are independently 1 to 10 and f is 1 to 50; and
m is 0 or 1, and n is 1 to 6.

Formula B represents those compounds in which
R⁹ is H, or an alkyl or alkyleneoxy group having 1 to 18 carbon atoms, or an allyl group, or an aryl or substituted aryl having the structure 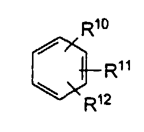 in which R¹⁰, R¹¹, and R¹² are independently H or an alkyl or alkyleneoxy group having 1 to 18 carbon atoms;
Z is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteroatom present may or may not be directly attached to K;
or Z is a urethane having the structure: 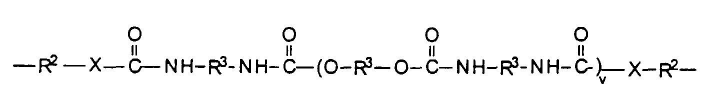 in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50;
or Z is an ester having the structure: 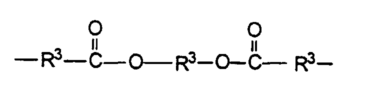 in which R³ is an alkyl or alkyloxy chain having up to 100 atoms in the chain, which chain may contain aryl substituents;
or Z is a siloxane having the structure:
   ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms and the R⁴ substituent independently for each position is an alkyl group having 1 to 5 carbon atoms or an aryl group, and e and g are independently 1 to 10 and f is 1 to 50;
K is an aromatic group selected from the aromatic groups having the structures (VI) through (XIII) (although only one bond may be shown to represent connection to the aromatic group K, this will be deemed to represent any number of additional bonds as described and defined by n): 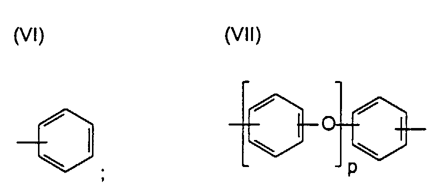 in which p is 1 to 100; 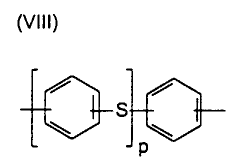 in which p is 1 to 100; 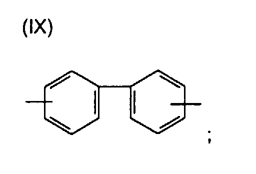 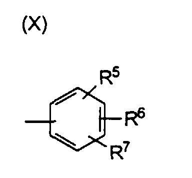 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteroatom present may or may not be directly attached to the aromatic ring; or R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 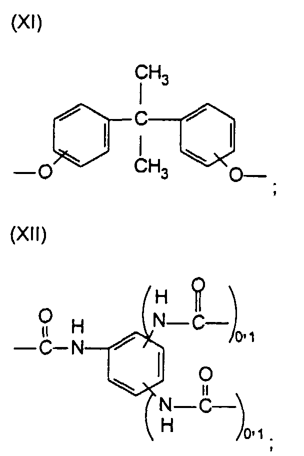 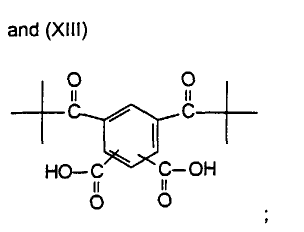
and m is 0 or 1 and n is 1 to 6.

### Vinyl Compounds

The compounds suitable for use in the adhesive compositions of this invention have a structure represented by one of the formulae:
[M―Xₘ]ₙ―Q or [M―Zₘ]ₙ―K, in which m is 0 or 1, and n is 1 to 6.

M represents a vinyl group and can be the maleimide moiety having the structure: 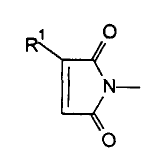 in which R¹ is H or C₁ to C₅ alkyl; or or the vinyl moiety having the structure: 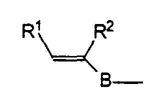 in which R¹ and R² are H or an alkyl having 1 to 5 carbon atoms, or together form a 5 to 9 membered ring with the carbons forming the vinyl group: B is C, S, N, O, C(O), O-C(O), C(O)-O, C(O)NH or C(O)N(R⁸), in which R⁸ is C₁ to C₅ alkyl. Preferably, B is O, C(O), O-C(O), C(O)-O, C(O)NH or C(O)N(R⁸); more preferably B is O, C(O), or C(O)N(R⁸).

X independently is an aromatic group selected from the aromatic groups having the structures (I) through (V): 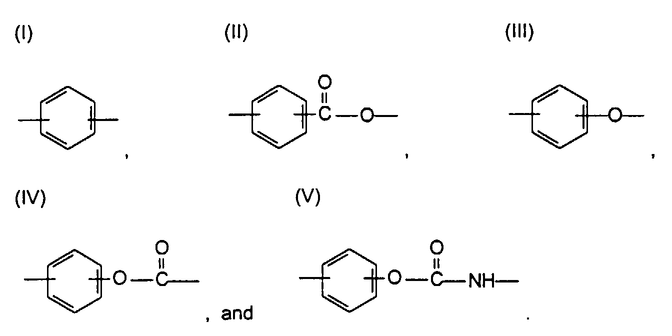 Preferably, X is structure (II), (III), (IV) or (V), and more preferably is structure (II).

Q and Z independently can be a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteroatom present may or may not be directly attached to X;
or Q and Z independently can be a urethane having the structure: 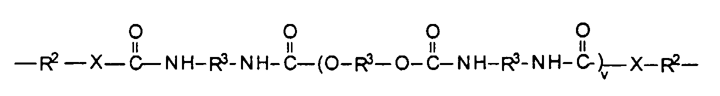 in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50;
or Q and Z independently can be an ester having the structure: 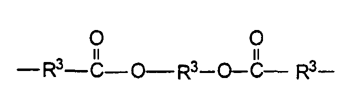 in which R³ is an alkyl or alkyloxy chain having up to 100 atoms in the chain, which chain may contain aryl substituents;
or Q and Z independently can be a siloxane having the structure: ―(CR¹₂)ₑ―[SiR⁴―O]_{f}―SiR⁴₂―(CR¹₂)_{g}― in which the R¹ substituent independently for each position is H or an alkyl group having 1 to 5 carbon atoms and the R⁴ substituent independently for each position is an alkyl group having 1 to 5 carbon atoms or an aryl group, and e and g are independently 1 to 10 and f is 1 to 50.

Preferably, Q and Z will be a linear or branched chain alkyl, alkyloxy, alkylene, or alkyleneoxy species having up to about 100 atoms in the chain, as described with pendant saturated or unsaturated cyclic or heterocyclic substituents, or a siloxane as described, and more preferably is a linear or branched chain alkyl species or siloxane, as described.

K is an aromatic group selected from the aromatic groups having the structures (VI) through (XIII) (although only one bond may be shown to represent connection to the aromatic group K, this will be deemed to represent any number of additional bonds as described and defined by n): 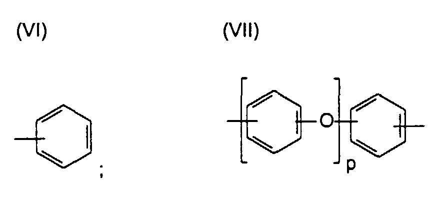 in which p is 1 to 100; 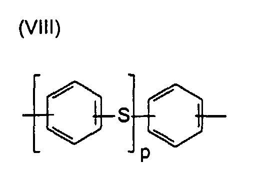 in which p is 1 to 100; 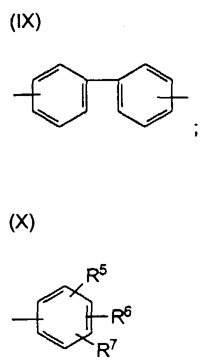 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteroatom present may or may not be directly attached to the aromatic ring; or R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50;

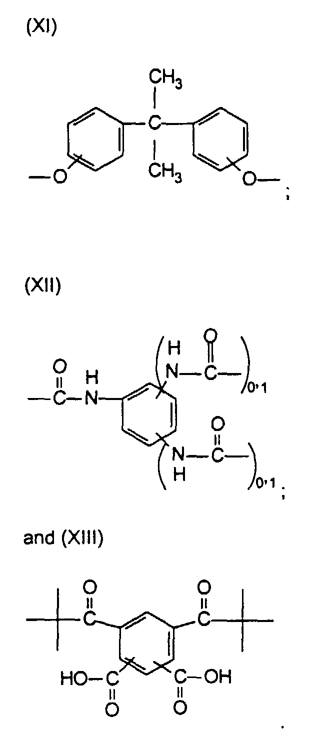

Preferably, K is structure (VIII), (X) or (XI), more preferably is structure (X) or (XI), and most preferably is structure (X).

### Other Composition Components

Depending on the nature of the substrate, the composition may also contain a coupling agent. A coupling agent as used herein is a chemical species containing a polymerizable functional group for reaction with the maleimide and other vinyl compound, and a functional group capable of condensing with metal hydroxides present on the surface of the substrate. Such coupling agents and the preferred amounts for use in compositions for particular substrates are known in the art. Suitable coupling agents are silanes, silicate esters, metal acrylates or methacrylates, titanates, and compounds containing a chelating ligand, such as phosphine, mercaptan, and acetoacetate. When present, coupling agents typically will be in amounts up to 10 percent by weight, and preferably in amounts of 0.1 to 3.0 percent by weight, of the allylated amide and vinyl compound, if any.

In addition, the compositions may contain compounds that lend additional flexibility and toughness to the resultant cured composition. Such compounds may be any thermoset or thermoplastic material having a Tg of 50°C or less, and typically will be a polymeric material characterized by free rotation about the chemical bonds, such as can be obtained by the presence of carbon-carbon double bonds adjacent to carbon-carbon single bonds, the presence of ester and ether groups, and the absence of ring structures. Suitable such modifiers include polyacrylates, poly(butadiene), polyTHF( polymerized tetrahydrofuran), CTBN (carboxy-terminated butyronitrile) rubber, and polypropylene glycol. When present, toughening compounds may be in an amount up to about 15 percent by weight of the maleimide and other monofunctional vinyl compound.

Siloxanes may also be added to the compositions to impart elastomeric properties. Suitable siloxanes are the methacryloxypropyl-terminated polydimethyl siloxanes, and the aminopropyl-terminated polydimethylsiloxanes, available from United Chemical Technologies.

The composition may also contain organic fillers, such as, polymers to adjust rheology. Other additives known and used in the art may also be used for specific purposes, such as, adhesion promoters. The selection of the types and amounts suitable is within the expertise of one skilled in the art.

### EXAMPLE 1

### Preparation of Bis Phenol F - Bis(diallylamide)

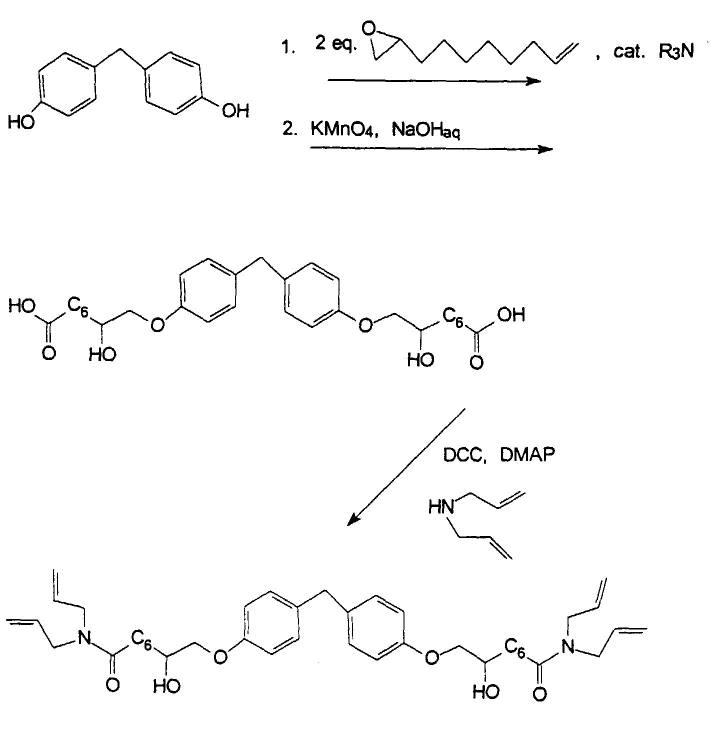

Bisphenol F (200.3 g, 1 mol) is solvated in tetrahydrofuran (THF) (500 mL) in a 2 L three-necked flask equipped with mechanical stirrer and reflux condenser. To this solution is added 1, 2-epoxy-9-decene (308.5 g, 2 mol) and benzyldimethylamine (0.67 g, 5 mmol). The solution is warmed to 80°C for 7 hours and then allowed to cool to room temperature. Solvent is removed *in vacuo* to yield an oil.

The intermediate isolated above (508.8 g, 1 mol) is dissolved in THF (1 L) and H₂O (1 L) in a 3 L three-necked flask equipped with mechanical stirrer, reflux condenser and internal temperature probe under nitrogen. To this solution is added KMnO4 (316 g, 2 mol), and the resulting mixture warmed to 80°C for 5 hours. The reaction is allowed to cool to room temperature and bulk solvent is removed *in vacuo*. The resulting material is solvated in CH₂Cl₂ (1 L), filtered, and washed with H₂O (3X1 L). The isolated organics are dried over MgSO₄ and solvent removed *in vacuo* to yield a diacid intermediate.

The above diacid (544.8 g, 1 mol) is combined with diallylamine (194.3 g, 2 mol) and CH₂Cl₂ (1 L) in a 3 L three-necked flask equipped with a mechanical stirrer, addition funnel and internal temperature probe under nitrogen. The solution is cooled to 4°C in an ice bath. The addition funnel is charged with dicyclohexylcarbodiimide (DCC) (412.7 g, 2 mol) dissolved in CH₂Cl₂ (300 mL), and this solution added to the stirred amine solution over the course of 60 minutes. The reaction is stirred on the ice bath for an additional 30 minutes. The mixture is allowed to warm to room temperature and further stirred for 4 hours. The solution is filtered to remove precipitated dicyclohexylurea (DCU). The isolated organics are dried over MgSO₄ anhyd., filtered and solvent removed *in vacuo* to yield the bis(diallylamide) product.

### EXAMPLE 2

### Preparation of Poly(butadiene)bis(diallylamide)

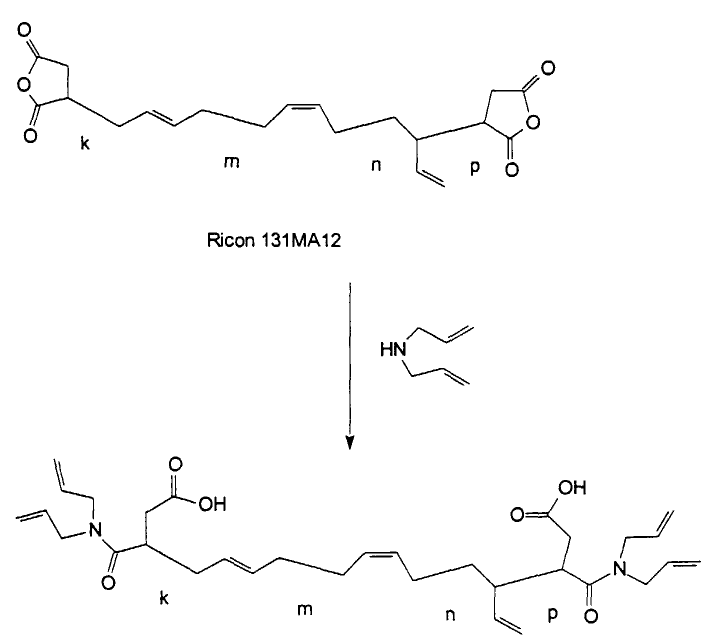

Diallylamine (97.15 g, 1mol) is solvated in acetone (500 mL) in a 2 L three-necked flask equipped with mechanical stirrer, addition funnel and internal temperature probe under nitrogen. The solution is cooled on an ice bath. Maleinized poly(butadiene) (Ricon 131MA5, Ricon Resins Inc., 1766 g) dissolved in acetone (500 mL) is charged into the addition funnel and added to the cooled amine solution over the course of 60 minutes maintaining an internal temperature <10°C. The solution is stirred on ice for an additional 60 minutes, then allowed to warm to room temperature and stirred for another 2 hours. Solvent and residual diallylamine are removed *in vacuo* to yield the poly(diallylamide).

### EXAMPLE 3

### Preparation of Dimer Bis(diallylamide) or 10,11-Dioctyl-1, 20-eicosyl bis(diallylamide)

Dimer acid (sold under the trademark Empol 1024 by Unichema, 20.5 g, 35.7 mmol) was solvated in anhydrous toluene (250 mL) in a 500 mL four-necked flask equipped with reflux condensor, addition funnel, and magnetic stirring under nitrogen. This solution was warmed to 80°C, and oxalyl chloride (12.5 mL, 143 mmol) was added dropwise over the course of 60 minutes. Evolution of CO₂, CO and HCl was immediately evident. The reaction was stirred for an additional 3 hours after the addition was complete, allowed to cool to room temperature and solvent removed *in vacuo* to yield an orange oil. IR and ¹H NMR spectral data were consistent with the desired bis(acid chloride) product.

Diallyl amine (10.0 mL) was solvated in diethyl ether (Et₂O) (200 mL) in a 500 mL three-necked flask equipped with mechanical stirrer, addition funnel and internal temperature probe under nitrogen. NaOH (3.2 g, 80 mmol) dissolved in H₂O (100 mL) was added to this solution. This solution was cooled to 4°C on an ice bath. The bis(acid chloride) described above was solvated in Et₂O (20 mL), charged into the addition funnel and added to the stirred amine solution over the course of 30 minutes, maintaining an internal temperature <10°C. This solution was stirred on ice for an additional one hour, then allowed to warm to room temperature, and stirred for an additional 4 hours. The organic layer was isolated and washed with 5% HCl_{aq} (200 mL), and H₂O (2x200mL). The isolated organics were dried over MgSO4 anhydrous, filtered, and the solvent removed *in vacuo* to yield an orange oil (87%), which exhibited IR and ¹H NMR spectral data consistent with the desired bis(diallylamide).

### EXAMPLE 4

### Preparation of Palmitoyl allylamide

Monoallylamide is prepared from allyl amine (57.1 g, 1mol), palmitoyl chloride (274.9 g, 1 mol) and NaOH (40 g, 1 mol) using the Schotten-Baumenn conditions described above for the synthesis of Example 3.

### EXAMPLE 5

### Allylamide/BMI Die Attach Adhesive

The following reagents were combined and manually mixed to yield a homogenous die attach composition:

| | |
|---|---|
| Azeloylbis(diallylamide) (prepared from azeloyl dichloride and diallylamine utilizing the same general procedure as in Example 3) | 0.521 g |
| Bismaleimide, sold as Versalink P-650 by Henkel | 1.678 g |
| *t*-Butyl-2-ethylhexanoate | 0.043 g |
| Metal diacrylate (sold as product number 633 by Sartomer) | 0.023 g |
| γ-Methacryloxypropyl-trimethoxysilane | 0.024 g |
| Silver powder from Chemet Corporation, RA-0081 | 5.148 g |

The adhesive was used to bond a 80 x 80 mil silicon die to various metal leadframes as listed below (Ag/Alloy 42 indicates silver coated Alloy 42, an Fe/Ni alloy; Ag/Cu indicates silver coated copper). Die Shear Strength was measured using an HMP Model 1750 die shear tester with digital force gauge DFI 50 (Chatillon) at room temperature (RDSS, room die shear strength) and at 240°C (HDSS, hot die shear strength) after curing for 60 seconds on a 200°C hotplate (condition 1), and after curing for 60 seconds on a 200°C hotplate followed by 4 hours in a 175°C oven (condition 2). The results are reported here and show commercially acceptable die shear values.

| Leadframe | Cure Condition 1 | | Cure Condition 2 | |
|---|---|---|---|---|
| | RDSS | HDSS | RDSS | HDSS |
| Ag/Alloy 42 | 1.72 | 0.87 | 5.65 | 0.92 |
| Ag/Cu | 2.15 | 0.58 | 4.65 | 0.74 |
| Cu | 1.76 | 0.48 | 3.60 | 0.85 |
| Pd | 1.94 | 1.18 | 4.56 | 1.25 |

### EXAMPLE 6

### Preparation of Benzamido-endcapped Dimer Diamine Bismaleimide

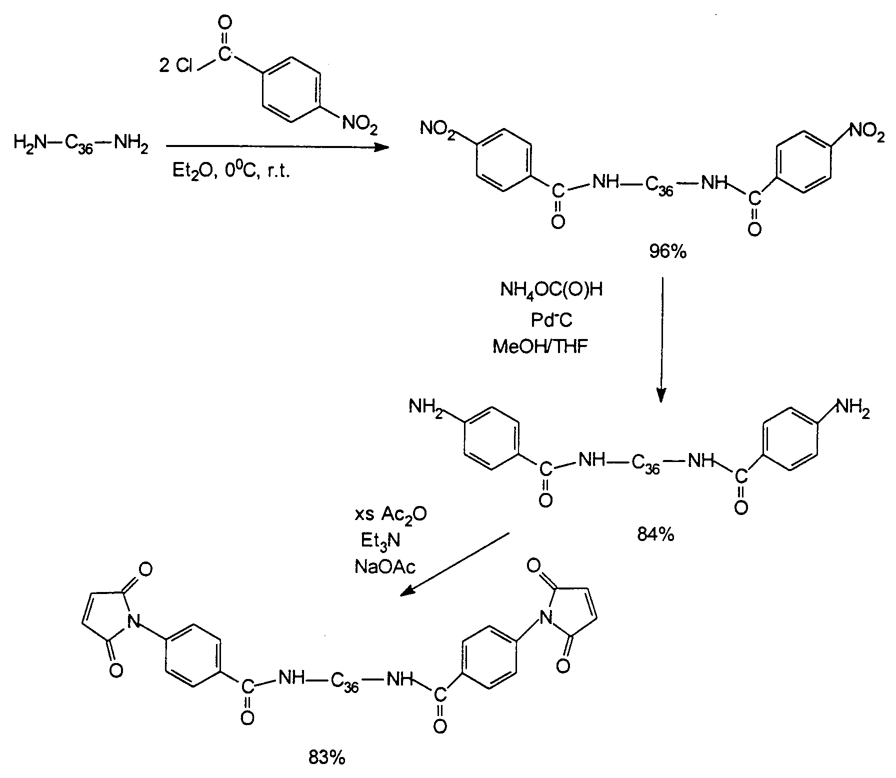

Dimer diamine (sold as Versamine 552 by Henkel, 20.0 g, 37 mmol) was solvated in diethyl ether (Et₂O) (200 mL) in a 500 mL three-necked flask equipped with an addition funnel, magnetic stirring, internal temperature probe and nitrogen inlet/outlet. NaOH_{aq} (11.7 mL of 6.25 M solution diluted with 100 mL H₂O, 73 mmol) was added with vigorous stirring. This solution was placed under a steady flow of nitrogen and cooled to 3°C on an ice bath with stirring. The addition funnel was charged with *p*-nitrobenzoyl chloride (13.6 g, 73 mmol) in Et₂O (50 mL), and this solution was added to the reaction vessel over the course of 60 minutes, maintaining an internal T<10°C. The reaction was stirred at ∼3°C for an additional 60 minutes after this addition was complete, then allowed to warm to room temperature and stirred for another 4 hours. The solution was transferred to a separatory funnel and the isolated organic layer washed with distilled H₂O (300 mL), 5% HCl_{aq} (300 mL), NaCl_{aq} (250 mL) and distilled H₂O (2x250 mL). The organics were isolated, dried over MgSO₄ anhyd., filtered and the solvent removed *in vacuo* to yield the dinitro compound as a viscous yellow oil which exhibited acceptable ¹H NMR and IR spectra (30.0 g, 96 %).

The dinitro compound described above (5.0 g, 5.9 mmol) was dissolved in methanol (MeOH) (25 mL) and tetrahydrofuran (THF) (5 mL) in a 250 mL three-necked flask equipped with magnetic stirring, reflux condensor and nitrogen inlet/outlet. The solution was placed under nitrogen, and 5% Pd-C (0.96 g) were added with stirring. Ammonium formate (3.4 g, 55 mmol) was added and the reaction stirred at room temperature for 2 hours. Carbon dioxide evolution was immediately observed. The reaction solution was filtered, and bulk filtrate solvent was removed via rotary evaporator. The resulting viscous oil was dissolved in Et₂O (150 mL), washed with distilled H₂O (150 mL), isolated and dried over MgSO₄ anhyd. Solvent was removed *in vacuo* to yield the diamine as a sticky tan oil, which exhibited acceptable ¹H NMR and IR spectra (3.9 g, 84%).

Maleic anhydride (0.5 g, 5.1 mmol) was dissolved in acetone (10 mL) in a 250 mL three-necked flask equipped with magnetic stirring, addition funnel and nitrogen inlet/outlet. The solution was cooled on an ice bath and placed under nitrogen. The addition funnel was charged with an acetone (10mL) solution of the diamine described above (2.0 g, 2.60 mmol), which was added dropwise over 30 minutes. The reaction was stirred for an additional 30 minutes on the ice bath, then allowed to warm to room temperature, and stirred for another 4 hours. To the resulting slurry was added acetic anhydride (Ac₂O) (1.54 mL, 160 mmol), triethyl amine (Et₃N) (0.23 mL, 1.63 mmol) and sodium acetate (NaOAc) (0.16 g, 1.9 mmol). The resulting slurry was heated to mild reflux for 5 hours. The reaction was allowed to cool to room temperature, and solvent removed *via* rotary evaporator to yield a brown oil. This material was dissolved in CH₂Cl₂ (250 mL) and washed with distilled H₂O (200mL), satd. NaHCO₃ (200 mL) and distilled H₂O (200 mL). Emulsions were broken by adding NaCl when necessary. The organic layer was isolated, dried over MgSO₄ anhyd. and solvent removed *in vacuo* to yield the bismaleimide, a brown solid (2.0 g, 83%). The resin exhibited satisfactory ¹H NMR, ¹³C NMR and IR spectra, which indicated slight contamination with acetic acid.

### EXAMPLE 7

### Preparation of 20-Bismaleimido-10,11-dioctyl-eicosame (and isomers)

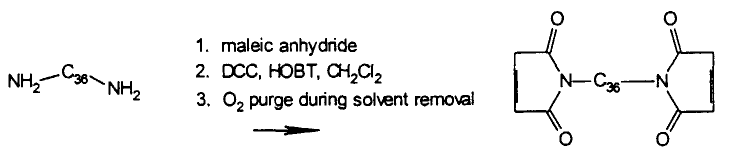

In a 5 L multi-neck flask equipped with a drying tube, thermometer, slow addition funnel , mechanical stirrer and nitrogen purge maleic anhydride (98.06 g, 1.02 equivalents on -NH₂) was dissolved in 500 ml tetrahydrofuran (THF). Stirring was begun and the solution was chilled with a dry ice/water bath. Slow addition of dimer diamine (Versamine 552, Henkel, 245.03 g, 0.4477 mol) in 250 ml THF was begun. Addition was carried out over 1 hour. After addition was complete the ice bath was removed and 375 ml of THF was rinsed through the slow addition funnel to incorporate solidified diamine. After one hour the ice bath was replaced around the flask. 1-Hydroxybenzotriazole (96.79 g, 0.80 equivalents on -NH₂) was added rinsing into the flask with 50ml THF. When the temperature had reached 5°C slow addition of dicyclohexylcarbodiimide (DCC) (188.43 g, 1.02 equivalents on -NH₂) in 200ml THF was begun. The temperature during addition was kept below ten degrees. After DCC addition was complete the slow addition funnel was rinsed with 80 ml of THF. The ice bath was removed. The reaction was monitored by IR. When it appeared that the isoimide has been converted to maleimide (approximately 4 hours after the completion of DCC addition) the mixture was filtered, rinsing the solids with THF. The orange solution was placed in the freezer overnight.

The solution was removed from the freezer and allowed to warm to room temperature. Hydroquinone (0.0513 g) was added to the solution. A partial strip of the THF was carried out on a rotary evaporator with the temperature maintained below 28°C. The solution was concentrated to approximately 800 ml. Much particulate matter was visible. The solution was placed in freezer overnight.

The mixture was removed from the freezer and allowed to warm. The solids were filtered, rinsing with THF. The filtrate was transferred to a 2 L multi-neck flask equipped with a mechanical stirrer, vacuum line connected to a trap, and a glass tube attached by tubing to a drying tube. The remaining THF was stripped at room temperature by pulling a vacuum and bubbling air through the material while stirring. The resultant thick, creamy-tan colored semi-solid was placed in the freezer overnight.

The semi-solid was removed from the freezer and allowed to warm. The semi-solid was dissolved in 450 ml each of methanol and hexane, and washed with 50% methanol/water (4 x 250 ml) to remove 1-hydroxybenzotriazole (HOBT). It was attempted to extract the product with hexane. After addition of 300 ml of hexane separation was not observed. The mixture was washed with additional water (3 x 250 ml). The organic phase was placed in the freezer overnight.

The material was removed from the freezer. Two layers were apparent. The upper layer was clear and yellow in color. The bottom layer was a orange and cloudy. The material was poured cold into a separatory funnel. The top layer was hexane and the desired product. The bottom layer was extracted with hexane (6 x 200 ml), separation occurred easily. The combined extracts were dried over anhydrous magnesium sulfate and filtered, rinsing the solids with hexane. The solvent was stripped to an approximate volume of 750 ml on a rotary evaporator with the temperature not exceeding 24°C. The remaining solvent was stripped off using a vacuum/air bubbling set-up at room temperature to give the desired product in 67% yield.

### EXAMPLE 8

### Preparation of Butadiene-Acrylonitrile Bismaleimide

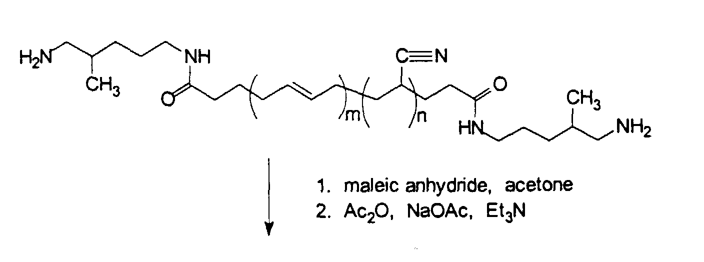

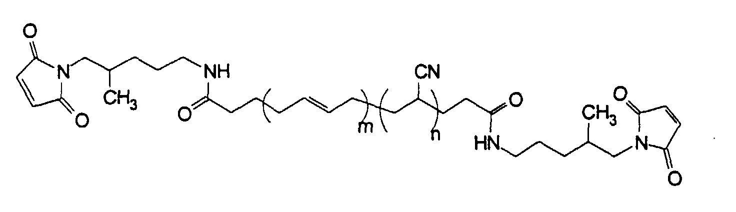

Amino-terminated butadiene-acrylonitrile (sold as Hycar resin 1300 X42 ATBN by BF Goodrich, in which the m and n depicted in the structure are integers to provide a number average molecular weight of 3600) (450 g, 500 mmol based on amine equivalent weight AEW = 450g) was dissolved in CHCl₃ (1000 mL) in a 3 L four-necked flask equipped with addition funnel, mechanical stirrer, internal temperature probe and nitrogen inlet/outlet. The stirred solution was placed under nitrogen and cooled on an ice bath. The addition funnel was charged with maleic anhydride (98.1 g, 1 mol) in CHCl₃ (50 mL) and this solution was added to the reaction over 30 minutes, maintaining the internal reaction temperature below 10°C. This mixture was stirred for 30 minutes on ice, then allowed to warm to room temperature and stirred for an additional 4hours. To the resulting slurry was added acetic anhydride (Ac₂O) (653.4 g, 6 mol), triethyl amine (Et₃N) (64.8 g, 0.64 mol) and sodium acetate (NaOAc) (62.3 g, 0.76 mol). The reaction was heated to mild reflux for 5 hours, allowed to cool to room temperature, and subsequently extracted with H₂O (1 L), satd. NaHCO₃ (1 L) and H₂O (2x1 L). Solvent was removed *in vacuo* to yield the maleimide terminated butadiene acrylonitrile.

### EXAMPLE 9

### Preparation of Tris(maleimide) Derived From Tris(epoxypropyl)isocyanurate

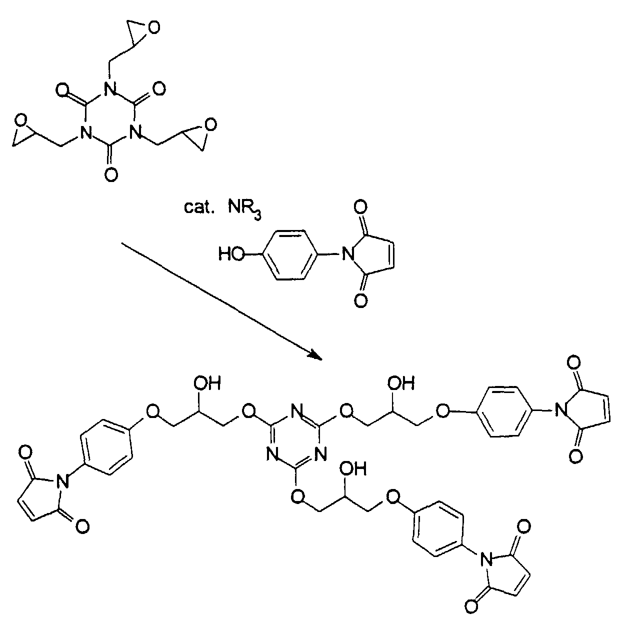

Tris(epoxypropyl)isocyanurate (99.0 g, 0.33 mol) iss dissolved in THF (500mL) in a 2 L three-necked flask equipped with mechanical stirrer, internal temperature probe and nitrogen inlet/outlet. To this solution is added hyroxyphenylmaleimide (189.2 g, 1 mol) and benzyldimethylamine (1.4 g, 0.05 wt. %). The solution is heated to 80°C for 7 hours. The reaction then is allowed to cool to room temperature, is filtered, and the filtrant washed with 5 % HCl_{aq} (500mL) and distilled H₂O (1 L). The resulting solid, triazinetris-(maleimide), is vacuum dried at room temperature.

### EXAMPLE 10

### Preparation of Maleimidoethylpalmitate

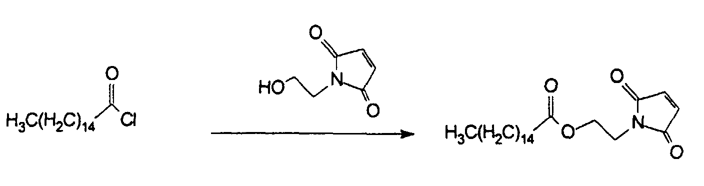

Palmitoyl chloride (274.9 g, 1 mol) is dissolved in Et₂O (500 mL) in a 2 L three-necked flask equipped with mechanical stirrer, internal temperature probe, addition funnel and nitrogen inlet/outlet. NaHCO₃ (84.0 g, 1 mol) in distilled H₂O (500 mL) is added with vigorous stirring and the solution cooled on an ice bath under nitrogen. The addition funnel is charged with hydroxyethylmaleimide (141 g, 1 mol) in Et₂O (100 mL) and this solution added to the reaction over a period of 30 minutes, maintaining an internal T<10°C during the addition. The reaction is stirred for another 30 minutes on ice, then allowed to warm to room temperature and stirred for 4 hours. The reaction is transferred to a separatory funnel and the isolated organic layer washed with distilled H₂O (500 mL), 5% HCl_{aq} (500 mL) and distilled H₂O (2x500 mL). The organics are isolated, dried over MgSO₄ anhyd., filtered and solvent removed *in vacuo* to yield the aliphatic maleimide.

### EXAMPLE 11

### Preparation of Bismaleimide Derived from 5-Isocyanato-1-(isocyanatomethyl)-1, 3, 3-trimethylcyclohexane

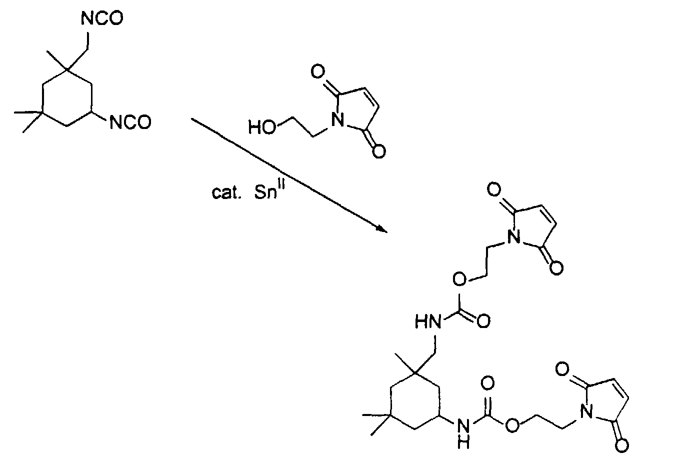

5-Isocyanato-1-(isocyanatomethyl)-1, 3, 3-trimethylcyclohexane (111.15 g, 0.5 mol) is solvated in THF (500 mL) in a 1 L three-necked flask equipped with mechanical stirrer, addition funnel and nitrogen inlet/outlet. The reaction is placed under nitrogen, and dibutyltin dilaurate (cat. Sn^{ll}) (6.31g, 10 mmol) and hydroxyethylmaleimide (141 g, 1 mol) are added with stirring, and the resulting mixture heated for 4 hours at 70°C. The addition funnel is charged with hydroxyethylmaleimide (141 g, 1 mol) dissolved in THF (100 mL). This solution is added to the isocyanate solution over 30 minutes, and the resulting mixture heated for an additional 4 hours at 70°C. The reaction is allowed to cool to room temperature and solvent removed *in vacuo*. The remaining oil is dissolved in CH₂Cl₂ (1 L) and washed with 10% HCl_{aq} (1 L) and distilled H₂O (2x1 L). The isolated organics are dried over MgSO₄, filtered and the solvent removed *in vacuo* to yield the maleimide.

### EXAMPLE 12

### Preparation of Dimer Divinyl Ether Derived From Pripol 2033

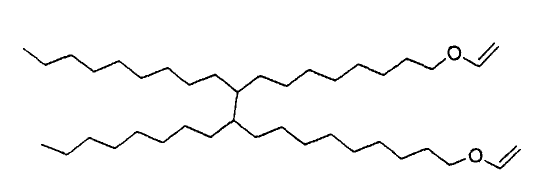

### "Dimer Divinyl Ether" (and cyclic isomers)

Bis(1, 10-phenanthroline)Pd(OAc)₂ (0.21 g, 0.54 mmol) was dissolved in a mixture of butyl vinyl ether (8.18 g, 81.7 mmols), heptane (100 mL) and "dimer diol" (sold as Pripol 2033 by Unichema, 15.4 g, 27.2 mmol) in 2 L three-necked flask equipped with a mechanical stirrer under nitrogen. This solution was heated to light reflux for 6 h. The solution was allowed to cool to room temperature and subsequently poured onto activated carbon (20 g) and stirred for 1 hour. The resulting slurry was filtered, and excess butyl vinyl ether and heptane were removed *in vacuo* to yield the divinyl ether as a yellow oil. The product exhibited acceptable ¹H NMR, FT-IR and ¹³C NMR spectral characteristics. Typical viscosity ∼100 cPs.

### EXAMPLE 13

### Preparation of Dimer Diacrylate Derived From Dimer Diol (Pripol 2033)

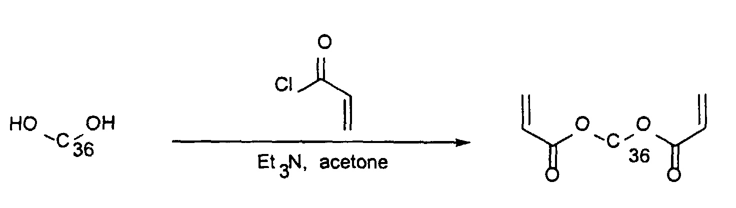

A dimer diol (sold as Pripol 2033 by Unichema, 284.4 g, 500 mmol) is dissolved in dry acetone (500 mL) in a 1 L three-necked flask equipped with mechanical stirrer, addition funnel and internal temperature probe under nitrogen. Triethylamine (101.2 g, 1 mol) is added to this solution and the solution cooled to 4°C on an ice bath. Acryloyl chloride (90.5 g, 1 mol) solvated in dry acetone (100 mL) is charged into the addition funnel and added to the stirred reaction solution over the course of 60 minutes, maintaining an internal temperature <10°C. This solution is stirred on ice for an additional 2 hours, then allowed to warm to room temperature and stirred for 4 hours. Bulk solvent is removed via a rotary evaporator, and the remaining residue solvated in CH₂Cl₂ (1 L). This solution is washed with 5% HCl_{aq} (800 mL), and H₂O (2x800mL). The isolated organics are dried over MgSO₄ anhyd and filtered, and the solvent removed *in vacuo* to yield the diacrylate as an oil.

### EXAMPLE 14

### Preparation of N-ethylphenyl Maleimide

4-Ethyl aniline (12.12g) was dissolved in 50 ml of anhydrous ethyl ether and slowly added to a stirred solution of 9.81 g of maleic anhydride in 100 ml of anhydrous ethyl ether chilled in an ice bath. After completion of the addition, the reaction mixture was stirred for 30 minutes. The light yellow crystals were filtered and dried. Acetic anhydride (200 ml) was used to dissolve the maleamic acid and 20 g of sodium acetate. The reaction mixture was heated in an oil bath at 160°C. After 3 hours of reflux, the solution was cooled to room temperature, placed in a 1L beaker in ice water and stirred vigorously for 1 hour. The product was suction-filtered and recrystallized in hexane. The collected crystalline material was dried at 50°C in a vacuum oven overnight. FTIR and NMR analysis showed the characteristics of ethyl maleimide.

### EXAMPLE 15

### Preparation of Bis(alkenylsulfide)

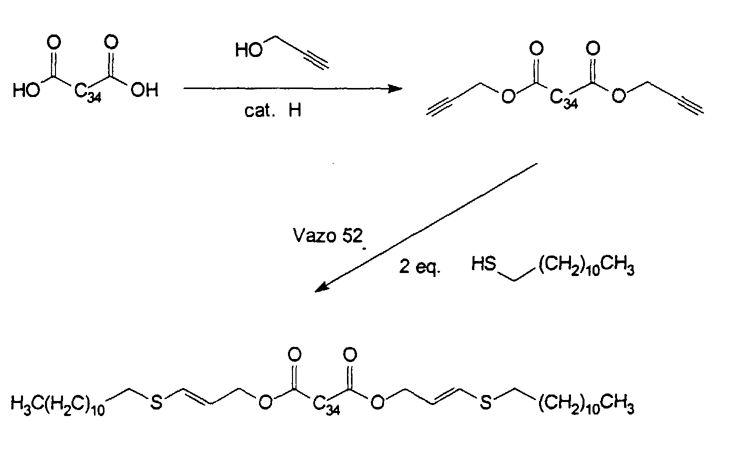

Dimer acid (sold under the trademark Empol 1024 by Unichema) (574.6 g, 1 mol) and propargyl alcohol (112.1 g, 2 mol) are solvated in toluene (1 L) in a 3 L three-necked flask equipped with mechanical stirring and a Dean-Stark distillation apparatus. Concentrated H₂SO₄ (6 mL) is added and the solution refluxed for 6 hours until 36 mL of H₂O is azeotropically distilled. The solution is allowed to cool to room temperature, is washed with H₂O (2X1L), dried over MgSO₄ anhyd and the solvent removed *in vacuo* to yield the propargyl ester intermediate as an oil.

This ester intermediate (650.7 g, 1 mol) is solvated in THF (200 mL) in a 1L three-necked flask equipped with reflux condensor, mechanical stirrer and internal temperature probe under nitrogen. Lauryl mercaptan (404.8 g, 2 mol) and 2,2'-azobis(2,4-dimethylpentanenitrile) (sold under the trademark Vazo 52 by DuPont) (11 g) are added and the resulting mixture heated to 70°C on an oil bath with stirring for 7 hours. The reaction is allowed to cool to room temperature and solvent removed *in vacuo* to yield the alkenyl sulfide as an oil.

### EXAMPLE A.

### Preparation of 6-maleimidocaproic acid

### 6-maleimidocaproic acid

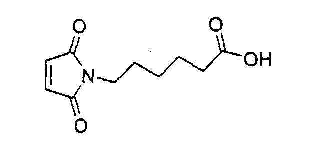

The acid functional maleimide, 6-maleimidocaproic acid, was synthesized using known methodology.¹ Aminocaproic acid (100 g, 7.6x10⁻¹ mols) was dissolved in glacial acetic acid (50 mL) in a 500 mL four-necked flask equipped with mechanical stirring, an internal temperature probe and an addition funnel. The addition funnel was charged with a solution of maleic anhydride (74.8 g, 7.6x10⁻¹ mols) dissolved in acetonitrile (75 mL). This solution was added to the aminocaproic acid at room temperature dropwise over 1 hour, maintaining an internal reaction temperature less than 35°C. The reaction was stirred for three hours after the addition was complete. The reaction slurry was filtered, and the isolated filtrate was dried in a vacuum oven (P∼25 T) overnight at 70°C to yield 166 g of off white solid (95%). The product amic acid exhibited FT-IR and ¹H NMR spectral characteristics consistent with literature data.

The amic acid described above (166 g, 7.2x10⁻¹ mols) was solvated in a solution of toluene (200 mL), benzene (200 mL) and triethylamine (211 mL, 1.51 mol) in a 1 L three-necked flask equipped with mechanical stirring and a Dean-Stark trap under nitrogen. This solution was heated to reflux for 4 h and the water produced collected in the Dean-Stark trap. Distilled water (400 mL) was added to the reaction flask to dissolve the triethylammonium salt of the product which largely separated from the bulk solution during the reaction. This aqueous layer was isolated, acidified to pH∼1 with 50% HCl, and extracted with ethyl acetate (600 mL). This organic layer was washed with distilled water (400 mL). The isolated organic layer was dried over MgSO₄, followed by solvent removal *in vacuo* to yield an off white solid (76.2 g, 50%). The product 6-maleimidocaproic acid was spectrographically identical to literature material by FT-IR and ¹H NMR.

### EXAMPLE B.

### Preparation of "Dimer Diester Bismaleimide"

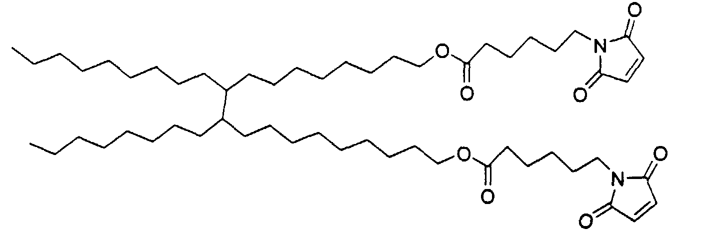

### "Dimer Diester Bismaleimide" (and cyclic isomers)

Pripol 2033 ("dimer diol", Uniqema, 92.4 g, 1.69x10⁻¹ mols), 6-maleimidocaproic acid (75.0 g, 3.55x10⁻¹ mols) and H₂SO₄ (0.50 mL, -8.5x10⁻³ mols) were slurried in toluene (300 mL) in a 1 L four-necked flask equipped with mechanical stirrer, a Dean-Stark trap and an internal temperature probe under nitrogen. The reaction was heated to light reflux for two hours and the water evolved collected in the Dean-Stark trap. The trap was drained and ∼50 mL of toluene solvent was distilled off of the reaction to remove trace moisture and drive the esterification equilibrium to completion. The reaction was allowed to cool to room temperature, additional toluene (100 mL) was added (on the laboratory scale it is preferable to add diethyl ether in place of toluene at this point), and the solution was washed with saturated NaHCO₃aq. (300 mL) and distilled water (300 mL). The organic layer was isolated and dried over anhydrous MgSO₄, and the solvent removed *in vacuo* to yield an orange oil (107.2 g, 68%). The material can be further purified by eluting a toluene solution of the resin through a short plug of silica or alumina. This liquid bismaleimide resin exhibited acceptable FT-IR, ¹H NMR, and ¹³C NMR data. Typical η∼2500 cPs.

### EXAMPLE C.

### Preparation of "Decane Dial Diester Bismaleimde"

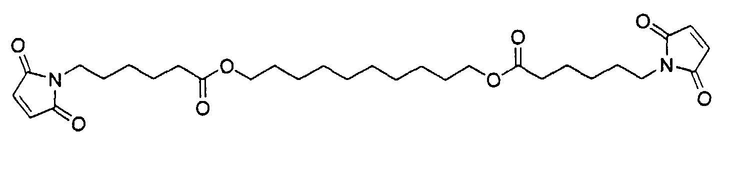

### "Decane Diol Diester Bismaleimide"

The general procedure described in Example B. was applied substituting decane diol (29.5 g, 1.69x10⁻¹ mols) for Pripol 2033. This process yielded a solid, moderately soluble bismaleimide (54.9 g, 58%). The product exhibited satisfactory FT-IR and ¹H NMR data.

### EXAMPLE D

### Preparation of "Glycerol Triester Tris(maleimide)"

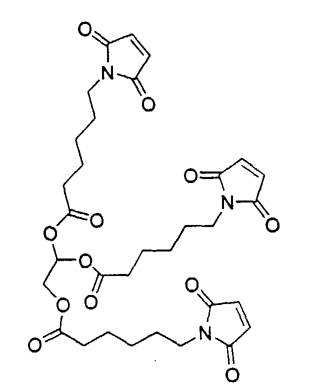

The protocol outlined in example B. was utilized substituting glycerol (10.4 g, 1.13x10⁻¹ mol) for Pripol 2033. The product was a viscous liquid which exhibited acceptable FT-IR and ¹H NMR data.

### EXAMPLE E.

### Preparation of "Bis(m-nitrobenzyl carbamate) of IPDI"

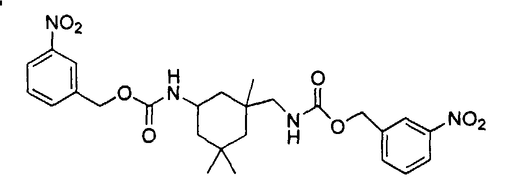

### "Bis(m-nitrobenzyl carbamate) of IPDI"

Isophorone diisocyanate ("IPDI", 100.0 g, 4.5x10⁻¹ mols), *m*-nitrobenzyl alcohol (137.8 g, 9.0x10⁻¹ mols) and dibutyl tin dilaurate (2.8 g, 4.5x10⁻³ mols) were solvated in dry toluene (1500 mL) in a 2L three-necked flask equipped with mechanical stirrer, reflux condensor and internal temperature probe under nitrogen. The resulting solution was heated to 90°C for 4 h. No isocyanate band was observed in the IR of the solids portion of the sample. The solution was allowed to cool to room temperature and washed with distilled H₂O (100 mL). The organic layer was isolated and solvent removed *in vacuo* to yield a yellow liquid which exhibited acceptable FT-IR and ¹H NMR characteristics.

### EXAMPLE F.

### Preparation of "Bis(m-aminobenzyl carbamate) of IPDI"

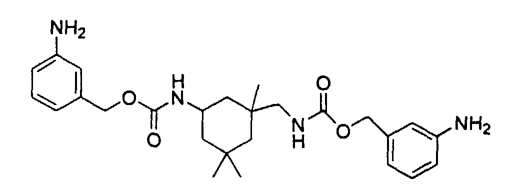

### "Bis(m-aminobenzyl carbamate) of IPDI"

The dinitro compound from Example E. (8.28 g, 1.57x10⁻² mols) was dissolved in ethanol (100 mL) in a 500 mL three-necked round bottom flask equipped with magnetic stirring under nitrogen. Cyclohexene (28.6 mL, 2.82x10⁻¹ mols) was added, followed by 5% Pd/C (4.14 g). The resulting slurry was refluxed lightly for 6.5 h. The FT-IR of a filtered aliquot of this solution exhibited no nitro stretching bands at 1529 cm⁻¹ and 1352 cm⁻¹. The bulk solution was allowed to cool to room temperature and filtered. Solvent was removed *in vacuo* to yield a yellow semisolid (6.6 g, 90%) which exhibited acceptable FT-IR and ¹H NMR spectral characteristics.

### EXAMPLE G.

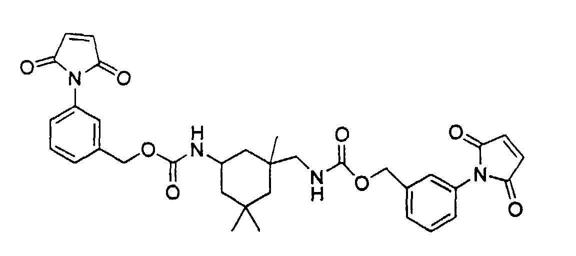

### Preparation of "Bis(m-maleimidobenzyl carbamate) of IPDI"

### "Bis(m-maleimidobenzyl carbamate) of IPDI"

The diamine from Example F (6.6 g, 1.41x1⁻² mols) was solvated in acetone (60 mL) in a 250 mL four-necked flask equipped with magnetic stirrer and addition funnel under nitrogen and cooled to 4°C. Maleic anhydride (2.76 g, 2.82x10⁻² mols) dissloved in acetone (20 mL) was added over the course of 30 minutes. The resulting solution was stirred at 4°C for for 1 h, and subsequently was allowed to warm to room temperature and stirred overnight. FT-IR analysis indicated no maleic anhydride remained as judged by the absence of the anhydride stretching band at ∼1810 cm⁻¹.

To the above amic acid solution was added acetic anhydride (8.5 mL, 9.0x10⁻² mols), triethylamine (1.26 mL, 9.0x10⁻³ mols) and sodium acetate (0.88 g, 1.1x10⁻² mols). The resulting solution was refluxed lightly for 4 h under nitrogen. The reaction was allowed to cool to room temperature and bulk solvent was removed *in vacuo*. The resulting viscous liquid was resolvated in methylene chloride (200 mL) and extracted with distilled water (3x200 mL). The organics were then dried over MgSO₄ anhyd., filtered and solvent removed *in vacuo* to yield a light brown solid (6.75 g, 76%). This material exhibited acceptable FT-IR and ¹H NMR spectral features.

### EXAMPLE H.

### Preparation of "Bis(m-nitrobenzyl carbamate) of DDI 1410"

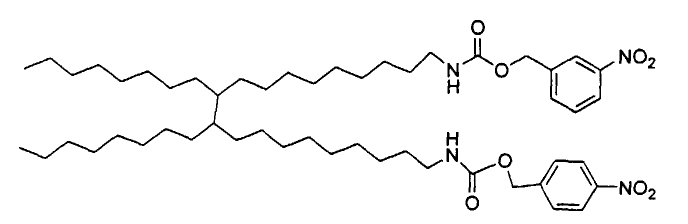

### "Bis(m-nitrobenzyl carbamate) of DDI 1410" (and cyclic isomers)

DDI 1410 (Henkel, "Dimer Diisocyanate", 99.77 g, 1.65x10⁻¹ mols based on 13.96 % NCO), *m*-nitrobenzyl alcohol (50.8 g, 3.32x10⁻¹ mols) and dibutyltin dilaurate (0.5 mL, 8.3x10⁻⁴ mols) were solvated in toluene (150 mL) in a 1 L four-necked flask equipped with mechanical stirrer, reflux condensor and internal temperature probe under nitrogen. The reaction was heated to 85°C for 2.5 h. FT-IR analysis of an aliquot of the reaction indicated complete comsumption of isocyanate functionality as judged by the lack of a band at 2272 cm⁻¹. Solvent was removed from the reaction in vacuo to yield a yellow oil which solidified upon standing at room temperature (152.4 g, 102% (trace toluene)). This solid exhibited satisfactory FT-IR and ¹H NMR spectral features.

### EXAMPLE I.

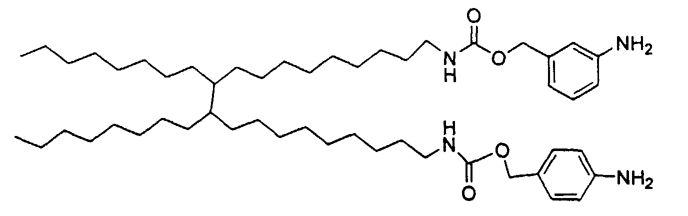

### Preparation of "Bis(m-aminobenzyl carbamate) of DDI 1410"

### "Bis(m-aminobenzyl carbamate) of DDI 1410" (and cyclic isomers)

The diamine product of Example H (39.6 g, 4.32x10⁻² mols) and stannous chloride dihydrate (97.55 g, 4.32x10⁻¹ mols) were slurried in ethyl acetate (300 mL) in a 1L three-necked flask equipped with mechanical stirrer and a reflux condensor under nitrogen. The reaction was heated to light reflux and stirred vigorously for 3 h. The solution was allowed to cool to room temperature and brought to pH 7-8 with a solution of saturated sodium bicarbonate. The mixture was pushed through a 25 micron filter to yield a mixture which separated into a cloudy aqueous layer and a moderately clear organic layer. The aqueous layer was isolated and washed with ethyl acetate (100 mL). The organic layers were combined, washed with distilled water (300 mL) and dried over anhydrous MgSO₄. The slurry was filtered and solvent removed from the filtrate *in vacuo* to yield yellow, sticky solid (33.8 g, 92%).

### EXAMPLE J.

### Preparation of "Bis(m-maleimidobenzyl carbamate) of DDI 1410"

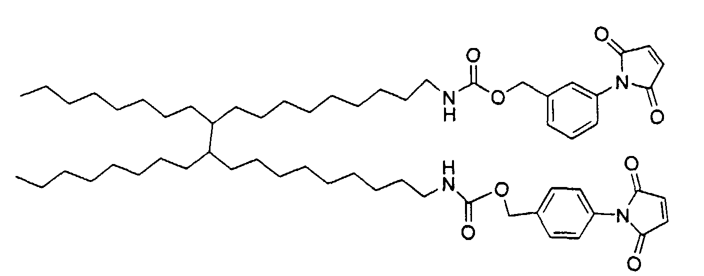

### "Bis(m-maleimidobenzyl carbamate) of DDI 1410" (and cyclic isomers)

Maleic anhydride (15.4 g, 1.57X10⁻² mols) was dissolved in acetone (300 mL) in a 2 L four-necked flask equipped with mechanical stirrer, internal temperature probe and addition funnel under nitrogen. This solutionn was cooled to ∼4°C on an ice bath. A solution of the diamine prepared in Example I (63.4 g, 7.48x10⁻² mols) in acetone (70 mL) was charged to the addition funnel and added to the maleic anhydride solution over a period of 30 minutes maintaining an internal temperature of <10°C. The resulting solution was stirred for 1 h and subsequently allowed to warm to room temperature and stir for 2 h.

To this solution of amic acid was added acetic anhydride (24.7 mL, 2.62x10⁻¹ mols), triethylamine (6.25 mL, 4.48x10⁻² mols) and manganese acetate tetrahydrate (0.37 g, 1.50x10⁻³ mols). This solution was heated to light reflux for 6.5 h, then allowed to cool to room temperature. Bulk solvent was removed *in vacuo*, and the resulting dark liquid was dissolved in diethyl ether (500 mL). This solution was washed with dist. H₂O (500 mL). The isolated organic layer was then washed with saturated NaHCO₃aq. (500 mL) and again with dist. H₂O (500 mL). The organics were isolated, dried over anhyd. MgSO₄, and solvent removed *in vacuo* to yield a viscous orange oil. This material exhibited FT-IR, ¹H NMR and ¹³C NMR spectral features consistent with the expected bismaleimide product.

Another embodiment of this invention includes the maleimides having the formulae [M―Xₘ]ₙ―Q and [M―Zₘ]ₙ―K as described herein in which Q and Z can be an ester having the structure 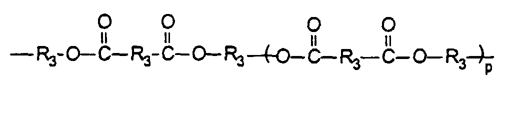 or the structure 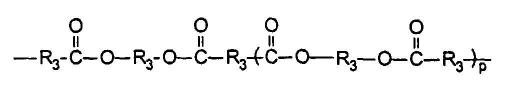
in which p is 1 to 100,
each R³ can independently be an alkyl or alkyloxy chain having up to 100 atoms in the chain, which chain may contain aryl substituents, or
a siloxane having the structure ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10 and f is 1 to 50.

Another embodiment of this invention includes the vinyl compounds having the structures 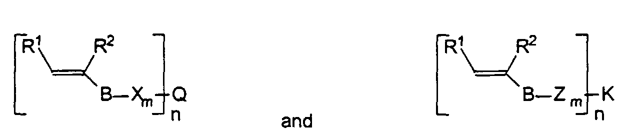 as described herein in which B is C, S, N, O, C(O), C(O)NH or C(O)N(R⁸), in which R⁸ is C₁ to C₅ alkyl.

Another embodiment of this invention includes the vinyl compounds having the structures 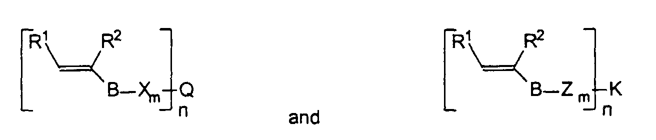
as described herein in which Q and Z can be an ester having the structure 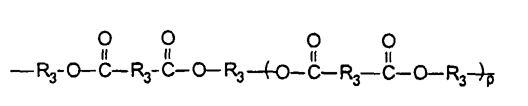 or the structure 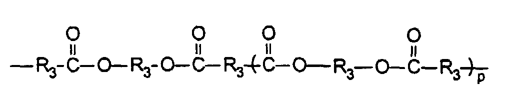
in which p is 1 to 100,
each R³ can independently be an alkyl or alkyloxy chain having up to 100 atoms in the chain, which chain may contain aryl substituents,
or a siloxane having the structure
   ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.

Another embodiment of this invention includes the curable adhesive composition as described herein containing an anionic or cationic curing initiator. The types and useful amounts of such initiators are well known in the art.

Other embodiments of this invention include those in the following numbered paragraphs:
1. An allylated amide compound having the formula 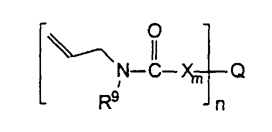 in which m is 0 or 1, n is 1 to 6, and
   (a) R⁹ is H, an alkyl group having 1 to 18 carbon atoms, an alkyleneoxy group having 1 to 18 carbon atoms, an allyl group, an aryl group, or a substituted aryl group having the structure 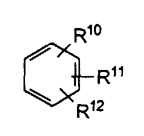 in which R¹⁰, R¹¹, and R¹² are independently H or an alkyl or alkyleneoxy group having 1 to 18 carbon atoms;
   (b) X is an aromatic group selected from the group of aromatic groups having the structures: 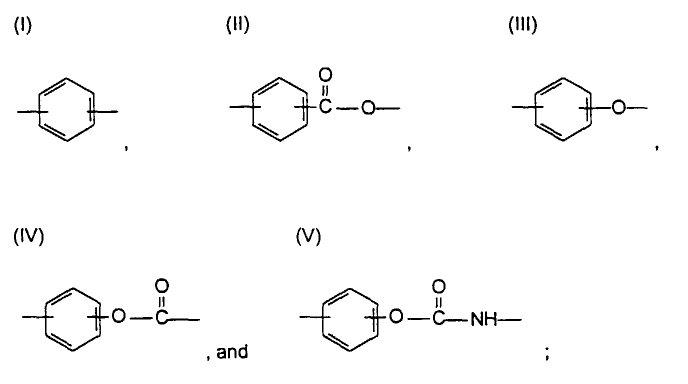
   (c) Q is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the chain, and in which any heteroatom present may or may not be directly attached to X.
2. The allylated amide compound according to paragraph 1 in which Q is a linear or branched chain alkyl species having up to about 100 atoms in the chain, which may contain saturated or unsaturated cyclic or heterocyclic substituents pendant from the chain or as part of the chain, and in which any heteroatom present may or may not be directly attached to X.
3. The allylated amide compound according to paragraph 1 in which Q is a urethane having the structure: 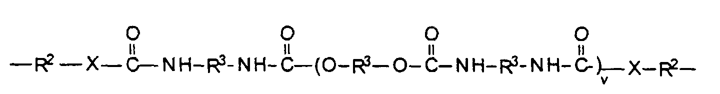
   in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50.
4. The allylated amide compound according to paragraph 1 in which Q is a siloxane having the structure (CR¹₂)ₑ―[SiR⁴₂―O]_{f}―SiR⁴₂―(CR¹₂)_{g}―
   in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
5. The allylated amide compound according to paragraph 1 in which Q is an ester having the structure: 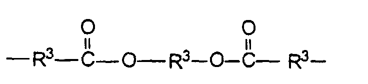
   in which R³ is an alkyl or alkyloxy chain having up to 100 atoms in the chain, which chain may contain aryl substituents.
6. The allylated amide compound according to paragraph 1 in which Q is an ester having the structure: 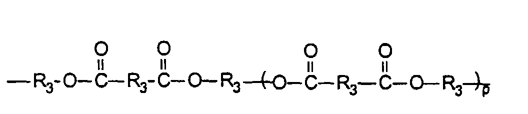
   in which p is 1 to 100,
   each R³ can independently be an alkyl or alkyloxy chain having up to 100 atoms in the chain, which chain may contain aryl substituents,
   or a siloxane having the structure
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
7. The allylated amide compound according to paragraph 1 in which Q is an ester having the structure: 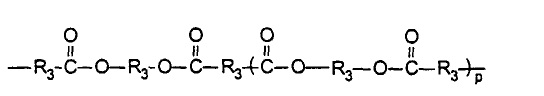
   in which p is 1 to 100,
   each R³ can independently be an alkyl or alkyloxy chain having up to 100 atoms in the chain, which chain may contain aryl substituents,
   or a siloxane having the structure
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
8. An allylated amide compound having the formula 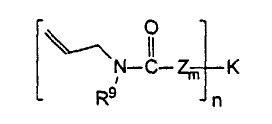 in which m is 0 or 1, n is 1-6, and
   (a) R⁹ is H, an alkyl or alkyleneoxy group having 1 to 18 carbon atoms, allyl, aryl, or substituted aryl having the structure 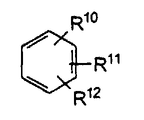 in which R¹⁰, R¹¹, and R¹² are independently H or an alkyl or alkyleneoxy group having 1 to 18 carbon atoms;
   (b) K is an aromatic group selected from group of aromatic groups having the structures: 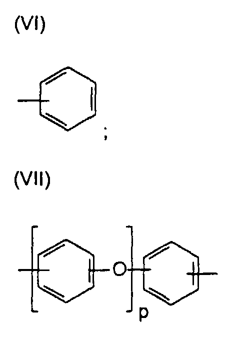 in which p is 1 to 100; 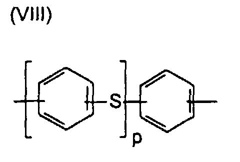 in which p is 1 to 100; 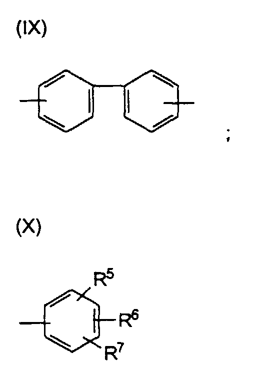 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteratom present may or may not be directly attached to the aromatic ring; or
      R⁵, R⁶, and R⁷ are a siloxane having the structure
         ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 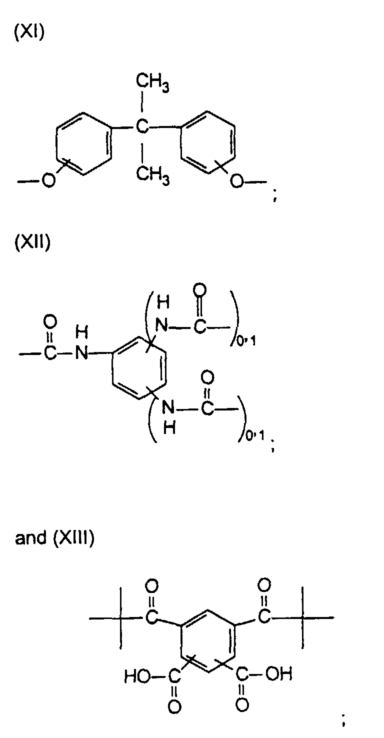
      and
   (c) Z is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the chain, and in which any heteroatom present may or may not be directly attached to K.
9. The allylated amide compound according to paragraph 8 in which Z is a linear or branched chain alkyl species having up to about 100 atoms in the chain, which may contain saturated or unsaturated cyclic or heterocyclic substituents pendant from the chain or as part of the chain, and in which any heteroatom present may or may not be directly attached to K.
10. The allylated amide compound according to paragraph 8 in which Z is a urethane having the structure: 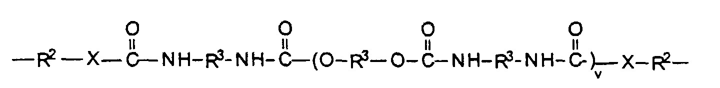
   in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50.
11. The allylated amide compound according to paragraph 8 in which Z is a siloxane having the structure: ―(CR¹₂)ₑ―[SiR⁴₂―O]_{f}―SiR⁴₂―(CR¹₂) _{g}―
   in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50.
12. The allylated amide compound according to paragraph 8 in which Z is an ester having the structure: 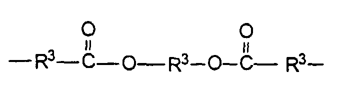
   in which R³ is an alkyl or alkyloxy chain having up to 100 atoms in the chain, which chain may contain aryl substituents.
13. The allylated amide compound according to paragraph 8 in which Z is an ester having the structure: 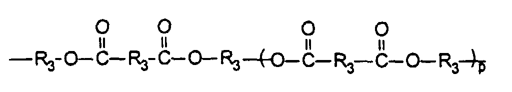
   in which p is 1 to 100,
   each R³ can independently be an alkyl or alkyloxy chain having up to 100 atoms in the chain, which chain may contain aryl substituents,
   or a siloxane having the structure
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
14. The allylated amide compound according to paragraph 8 in which Z is an ester having the structure: 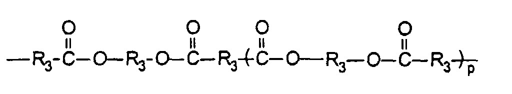
   in which p is 1 to 100,
   each R³ can independently be an alkyl or alkyloxy chain having up to 100 atoms in the chain, which chain may contain aryl substituents,
   or a siloxane having the structure
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
15. The allylated amide compound according to any one of paragraphs 8 to 14 in which K is 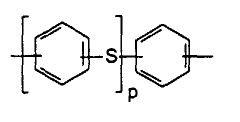 in which p is 1 to 100.
16. The allylated amide compound according to any one of paragraphs 8 to 14 in which K is 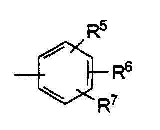 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the chain, and in which any heteroatom present may or may not be directly attached to the aromatic ring.
17. The allylated amide compound according to any one of paragraphs 8 to 14 in which K is 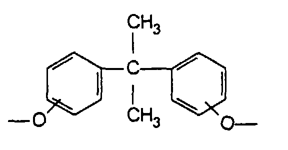
18. A curable adhesive composition comprising an allylated amide compound according to any one of paragraphs 1 through 17, a curing initiator selected from the group consisting of a free-radical initiator and a photoinitiator, optionally a filler and optionally an adhesion promoter.
19. A curable adhesive composition comprising an allylated amide compound according to any one of paragraphs 1 through 17 a curing initiator selected from the group consisting of an anionic and a cationic initiator, optionally a filler, and optionally an adhesion promoter.
20 A curable adhesive composition according to paragraph 18, further comprising a compound having the structure
   [M―Xₘ]ₙ― Q in which m is 0 or 1 and n is 1 to 6, and
   (a) M is a maleimide moiety having the structure: 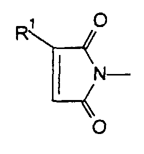 in which R¹ is H or an alkyl group having 1 to 5 carbon atoms;
   (b) X is an aromatic group selected from the group of aromatic groups having the structures: 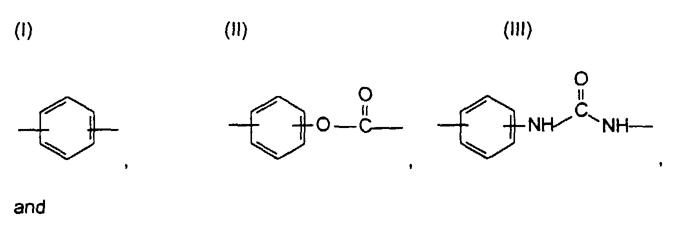 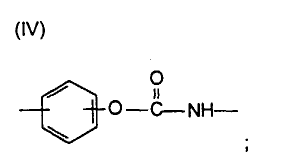 and
   (c) Q is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to X; or
   (d) Q is a urethane having the structure: 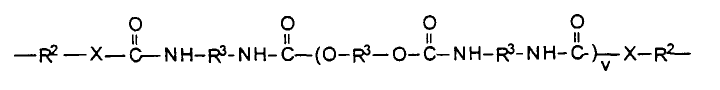
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (e) Q is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (f) Q is an ester having the structure: 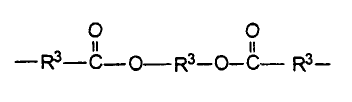
      in which R³ is an alkyl or alkyloxy chain having up to 100 atoms in the chain, which chain may contain aryl substituentsl; or
   (g) Q is an ester having the structure: 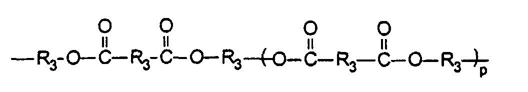
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (h) Q is an ester having the structure: 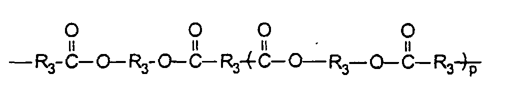
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
21. A curable adhesive composition according to paragraph 18 further comprising a compound having the structure 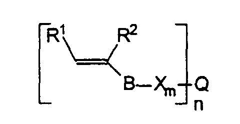 in which m is 0 or 1 and n is 1-6; and
   (a) R¹ and R² are H or an alkyl group having 1 to 5 carbon atoms, or together form a 5 to 9 membered ring with the carbons forming the vinyl group;
   (b) B is C, S, N, O, C(O), C(O)NH or C(O)N(R⁸), in which R⁸ is an alkyl group having 1 to 5 carbon atoms;
   (c) X is an aromatic group selected from the group of aromatic groups having the structures: 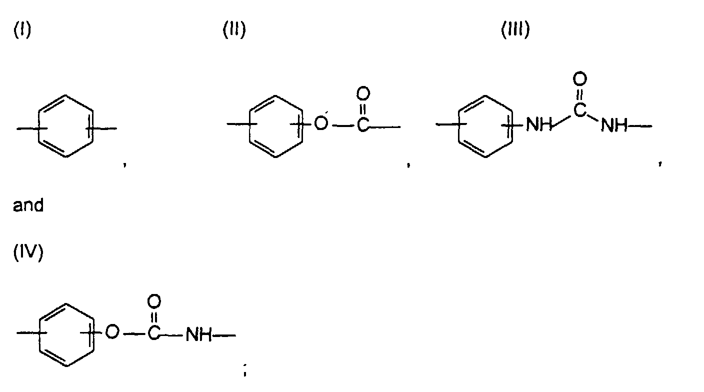 and
   (d) Q is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to X; or
   (e) Q is a urethane having the structure: 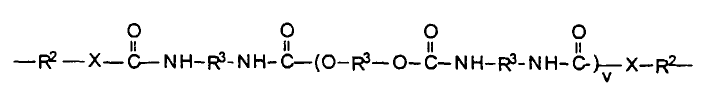
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (f) Q is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (g) Q is an ester having the structure: 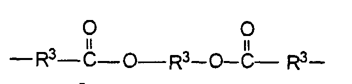
      in which R³ is an alkyl or alkyloxy chain having up to 100 atoms in the chain, which chain may contain aryl substituentsl; or
   (h) Q is an ester having the structure: 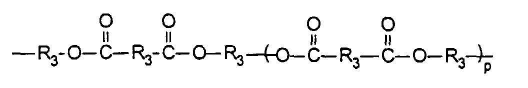
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (i) Q is an ester having the structure: 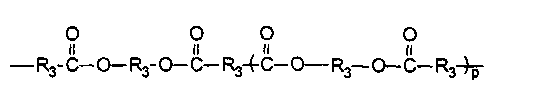
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
22. A curable adhesive composition according to paragraph 18 further comprising a compound having the structure
   [M―Zₘ]ₙ― K in which m is 0 or 1 and n is 1-6, and
   (a) M is a maleimide moiety having the structure 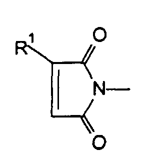 in which R¹ is H or an alkyl having 1 to 5 carbon atoms;
   (b) K is an aromatic group selected from group of aromatic groups having the structures: 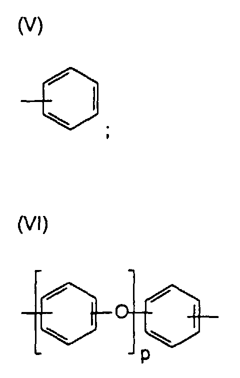 in which p is 1 to 100; 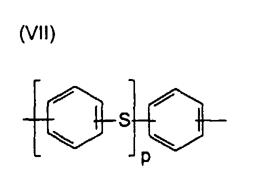 in which p is 1 to 100; 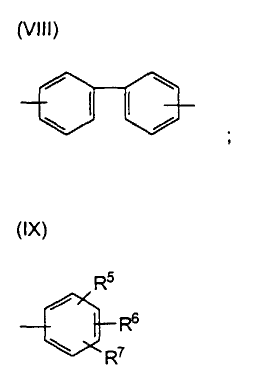 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the backbone in the chain, and in which any heteratom present may or may not be directly attached to the aromatic ring;
      or R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 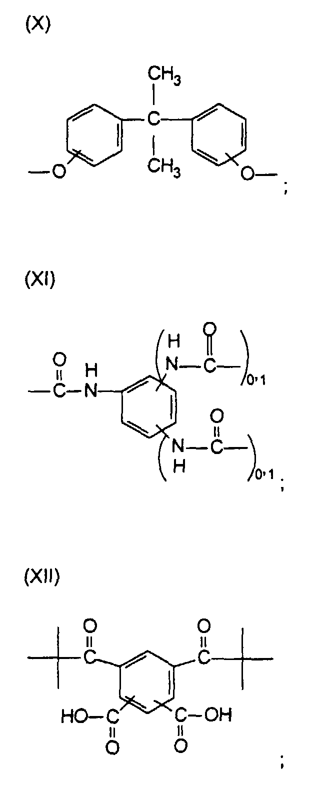
      and
   (c) Z is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to K; or
   (d) Z is a urethane having the structure: 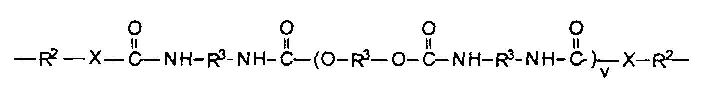
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (e) Z is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (f) Z is an ester having the structure: 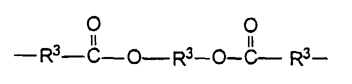
      in which R³ is an alkyl or alkyloxy chain having up to 100 atoms in the chain, which chain may contain aryl substituentsl; or
   (g) Z is an ester having the structure: 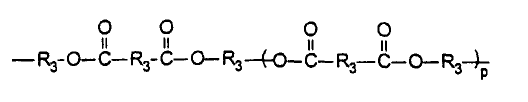
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (h) Z is an ester having the structure: 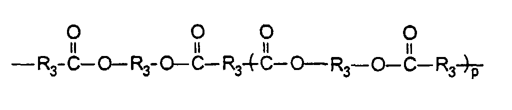
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
23. A curable adhesive composition according to paragraph 18 further comprising a compound having the structure 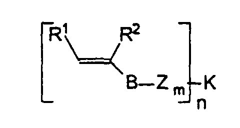 in which m is 0 or 1 and n is 1-6; and
   (a) R¹ and R² are H or an alkyl group having 1 to 5 carbon atoms, or together form a 5 to 9 membered ring with the carbons forming the vinyl group;
   (b) B is C, S, N, O, C(O), C(O)NH or C(O)N(R⁸), in which R⁸ is an alkyl group having 1 to 5 carbon atoms;
   (c) K is an aromatic group selected from the group of aromatic groups having the structures: 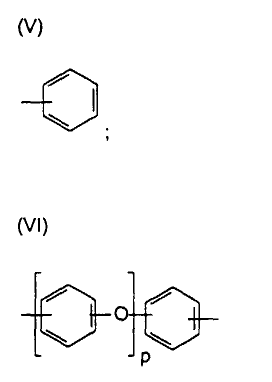 in which p is 1 to 100; 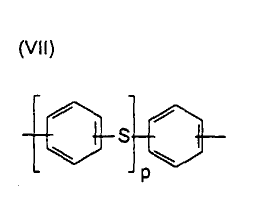 in which p is 1 to 100; 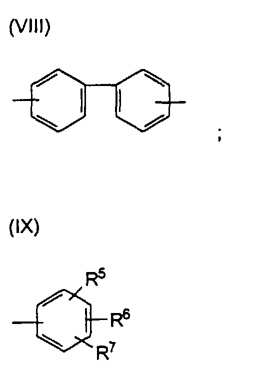 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the backbone in the chain, and in which any heteratom present may or may not be directly attached to the aromatic ring; or
      R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 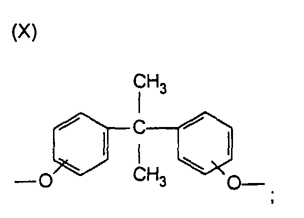 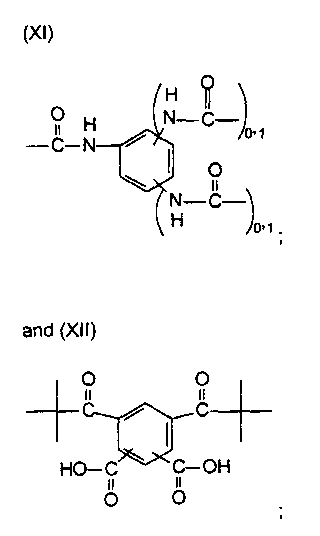
      and
   (d) Z is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to K; or
   (e) Z is a urethane having the structure: 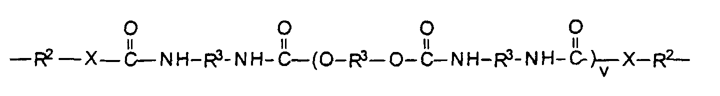
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (f) Z is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (g) Z is an ester having the structure: 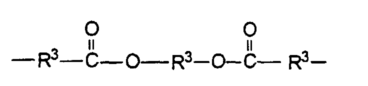
      in which R³ is an alkyl or alkyloxy chain having up to 100 atoms in the chain, which chain may contain aryl substituents; or
   (h) Z is an ester having the structure: 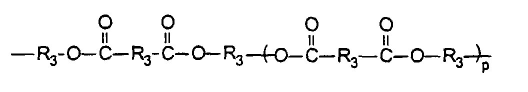
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (i) Z is an ester having the structure: 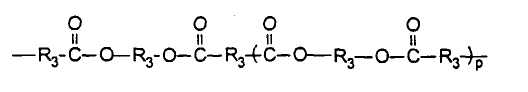
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and F is 1 to 50.
24. A curable adhesive composition according to paragraph 19 further comprising a compound having the structure
   [M―Xₘ]ₙ― Q in which m is 0 or 1 and n is 1 to 6, and
   (a) M is a maleimide moiety having the structure: 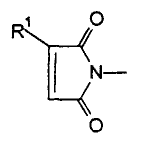 in which R¹ is H or an alkyl group having 1 to 5 carbon atoms;
   (b) X is an aromatic group selected from the group of aromatic groups having the structures: 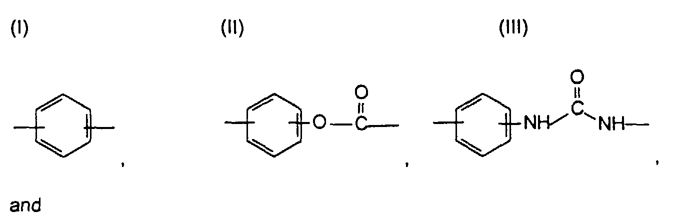 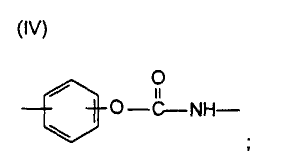 and
   (c) Q is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to X; or
   (d) Q is a urethane having the structure: 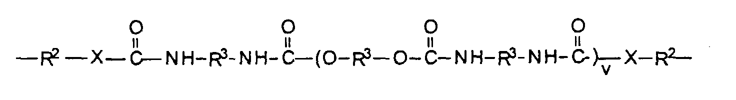
      in which each R² independently is an alkyl, aryl, or arylalkyl group having 1 to 18 carbon atoms; R³ is an alkyl or alkyloxy chain having up to 100 atoms in the chain, which chain may contain awl substituents; X is O, S, N, or P; and v is 0 to 50; or
   (e) Q is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (f) Q is an ester having the structure: 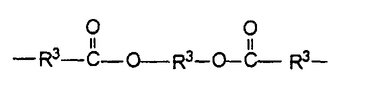
      in which R³ is an alkyl or alkyloxy chain having up to 100 atoms in the chain, which chain may contain aryl substituentsl; or
   (g) Q is an ester having the structure: 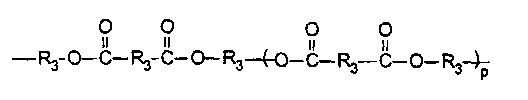
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (h) Q is an ester having the structure: 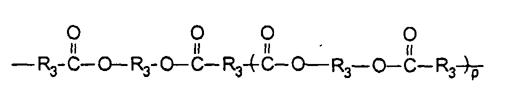
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
25. A curable adhesive composition according to paragraph 19 further comprising a compound having the structure 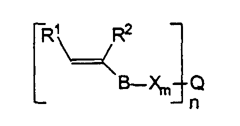 in which m is 0 or 1 and n is 1-6; and
   (a) R¹ and R² are H or an alkyl group having 1 to 5 carbon atoms, or together form a 5 to 9 membered ring with the carbons forming the vinyl group;
   (b) B is C, S, N, O, C(O), C(O)NH or C(O)N(R⁸), in which R⁸ is an alkyl group having 1 to 5 carbon atoms;
   (c) X is an aromatic group selected from the group of aromatic groups having the structures: 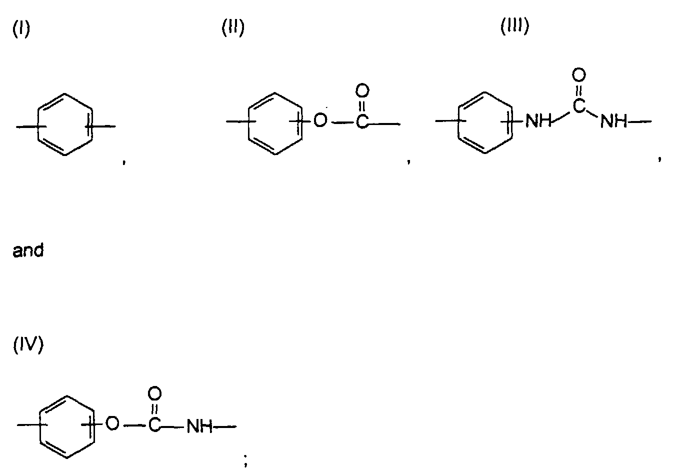 and
   (d) Q is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to X; or
   (e) Q is a urethane having the structure: 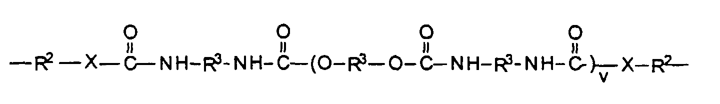
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (f) Q is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (g) Q is an ester having the structure: 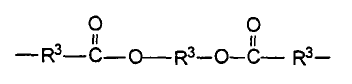
      in which R³ is an alkyl or alkyloxy chain having up to 100 atoms in the chain, which chain may contain aryl substituentsl; or
   (h) Q is an ester having the structure: 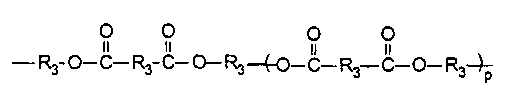
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (i) Q is an ester having the structure: 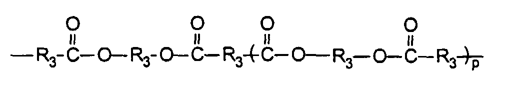
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
26. A curable adhesive composition according to paragraph 19 further comprising a compound having the structure
   [M―Zₘ]ₙ―K in which m is 0 or 1 and n is 1-6, and
   (a) M is a maleimide moiety having the structure 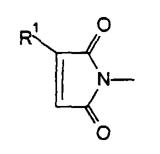 in which R¹ is H or an alkyl having 1 to 5 carbon atoms;
   (b) K is an aromatic group selected from group of aromatic groups having the structures: 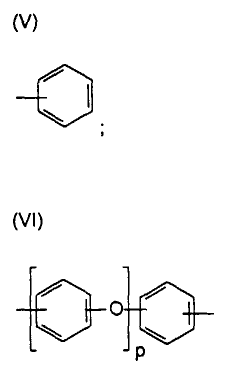 in which p is 1 to 100; 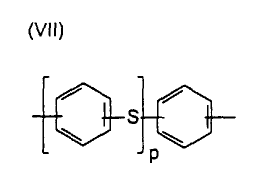 in which p is 1 to 100; 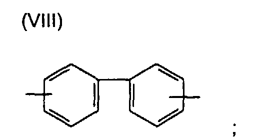 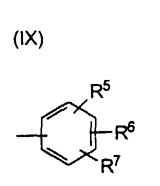 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the backbone in the chain, and in which any heteratom present may or may not be directly attached to the aromatic ring;
      or R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 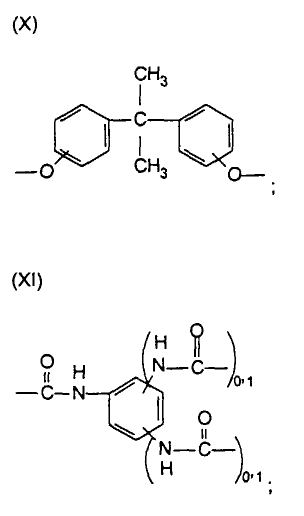 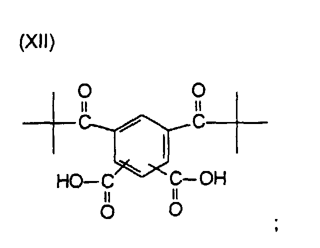
      and
   (c) Z is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to K; or
   (d) Z is a urethane having the structure: 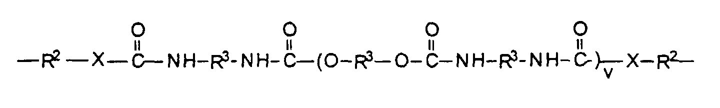
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (e) Z is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (f) Z is an ester having the structure: 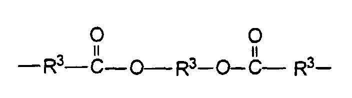
      in which R³ is an alkyl or alkyloxy chain having up to 100 atoms in the chain, which chain may contain aryl substituentsl; or
   (g) Z is an ester having the structure: 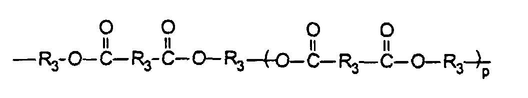
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (h) Z is an ester having the structure: 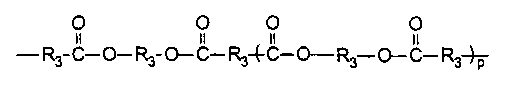
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
27. A curable adhesive composition according to paragraph 19 further comprising a compound having the structure 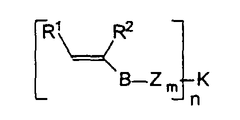 in which m is 0 or 1 and n is 1-6; and
   (a) R¹ and R² are H or an alkyl group having 1 to 5 carbon atoms, or together form a 5 to 9 membered ring with the carbons forming the vinyl group;
   (b) B is C, S, N, O, C(O), C(O)NH or C(O)N(R⁸), in which R⁸ is an alkyl group having 1 to 5 carbon atoms;
   (c) K is an aromatic group selected from the group of aromatic groups having the structures: 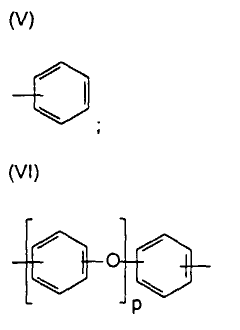 in which p is 1 to 100; 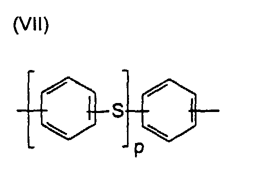 in which p is 1 to 100; 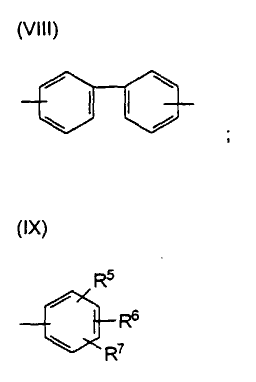 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the backbone in the chain, and in which any heteratom present may or may not be directly attached to the aromatic ring; or
      R⁵, R⁶, and R⁷ are a siloxane having the structure ―(CR¹₂)ₑ―[SiR⁴₂―O]_{f}―SiR⁴₂―(CR¹₂)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 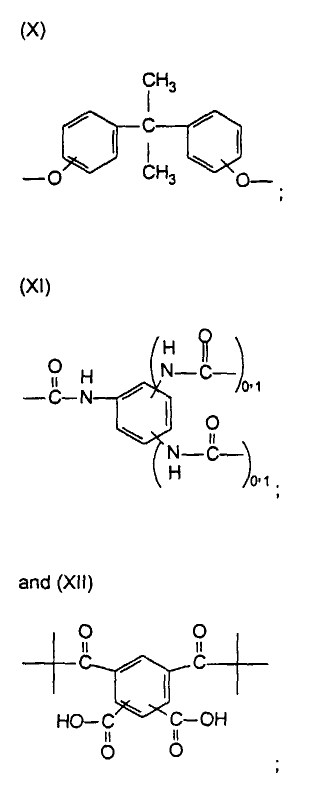
      and
   (d) Z is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to K; or
   (e) Z is a urethane having the structure: 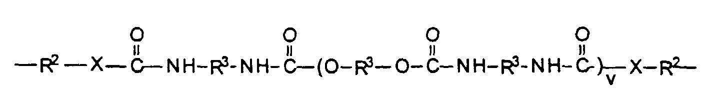
      in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
   (f) Z is a siloxane having the structure:
      ―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
   (g) Z is an ester having the structure: 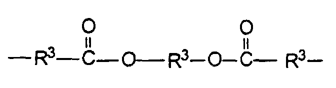
      in which R³ is an alkyl or alkyloxy chain having up to 100 atoms in the chain, which chain may contain aryl substituents; or
   (h) Z is an ester having the structure: 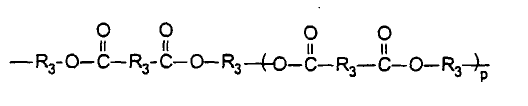
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
   (i) Z is an ester having the structure: 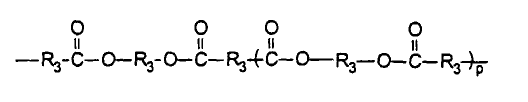
      in which p is 1 to 100, and
      each R³ can independently be an alkyl or alkyloxy chain having up to 100 atoms in the chain, which chain may contain aryl substituents; or
      each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.
28. A method for preparing an electronic assembly comprising an electronic component adhered to a substrate comprising
   (a) providing a curable adhesive according to any one of paragraphs 1 through 27,
   (b) applying the adhesive to the component or the substrate,
   (c) contacting the component and the substrate, and
   (d) curing the adhesive in situ.
29. An electronic assembly prepared by the method according to paragraph 28.

## Claims

1. An allylated amide compound having the formula 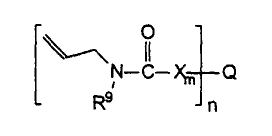 in which m is 0 or 1, n is 1 to 6, and
(a) R⁹ is H, an alkyl group having 1 to 18 carbon atoms, an alkyleneoxy group having 1 to 18 carbon atoms, an allyl group, an aryl group, or a substituted aryl group having the structure 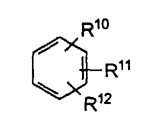 in which R¹⁰, R¹¹, and R¹² are independently H or an alkyl or alkyleneoxy group having 1 to 18 carbon atoms;
(b) X is an aromatic group selected from the group of aromatic groups having the structures: 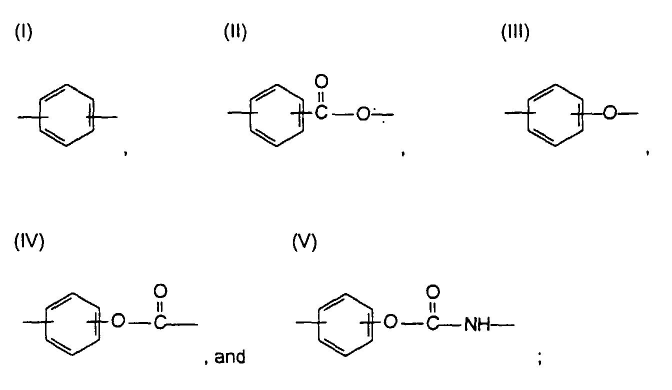
(c) Q is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the chain, and in which any heteroatom present may or may not be directly attached to X; or
(d) Q is a urethane having the structure: 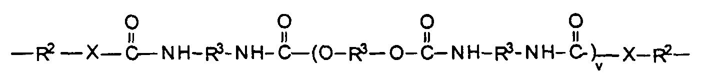
in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
(e) Q is a siloxane having the structure (CR¹₂)ₑ―[SiR⁴₂―O]_{f}― SiR⁴₂―(CR¹₂)_{g}―
in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
(f) Q is an ester having the structure: 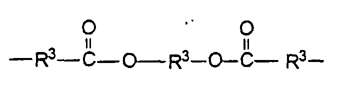
in which R³ is an alkyl or alkyloxy chain having up to 100 atoms in the chain, which chain may contain aryl substituents.
(g) Q is an ester having the structure: 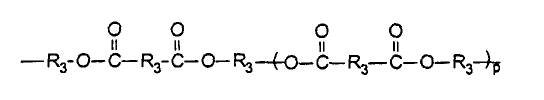
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
(h) Q is an ester having the structure: 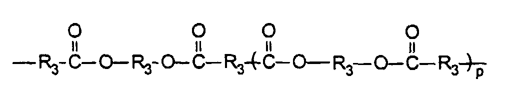
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.

2. An allylated amide compound having the formula 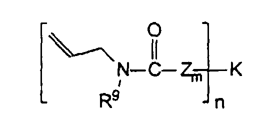 in which m is 0 or 1, n is 1-6, and
(a) R⁹ is H, an alkyl or alkyleneoxy group having 1 to 18 carbon atoms, allyl, aryl, or substituted aryl having the structure 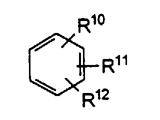 in which R¹⁰, R¹¹, and R¹² are independently H or an alkyl or alkyleneoxy group having 1 to 18 carbon atoms;
(b) K is an aromatic group selected from group of aromatic groups having the structures: 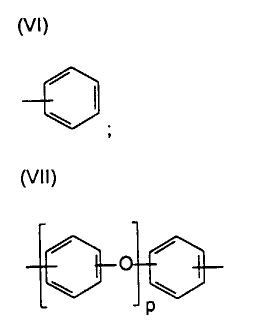 in which p is 1 to 100; 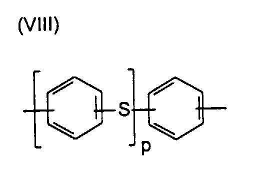 in which p is 1 to 100; 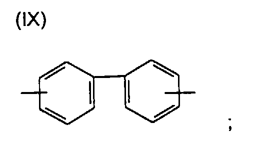 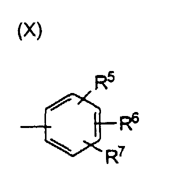 in which R⁵, R⁶ and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the backbone in the chain, and in which any heteratom present may or may not be directly attached to the aromatic ring; or
R⁵, R⁶, and R⁷ are a siloxane having the structure
―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 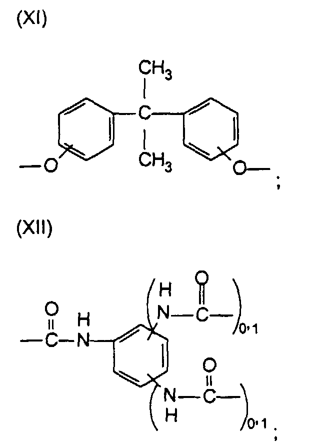 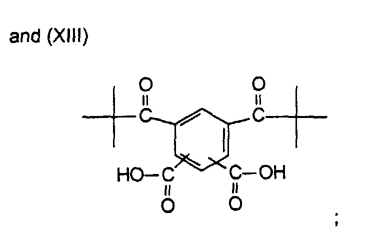
and
(c) Z is a linear or branched chain alkyl, alkyloxy, alkyl amine, alkyl sulfide, alkylene, alkyleneoxy, alkylene amine, alkylene sulfide, aryl, aryloxy, or aryl sulfide species having up to about 100 atoms in the chain, which may contain saturated or unsaturated cyclic or heterocyclic substituents pendant from the chain or as part of the chain, and in which any heteroatom present may or may not be directly attached to K; or
(d) Z is a urethane having the structure: 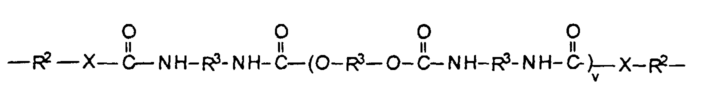
in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
(e) Z is a siloxane having the structure: ―(CR¹₂)ₑ―[SiR⁴₂―O]_{f}― SiR⁴₂―(CR¹₂)_{g}―
in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
(f) Z is an ester having the structure: 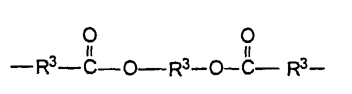
in which R³ is an alkyl or alkyloxy chain having up to 100 atoms in the chain, which chain may contain aryl substituents; or
(g) Z is an ester having the structure: 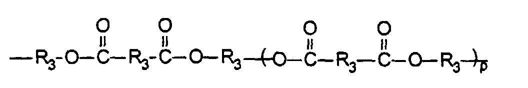
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
(h) Z is an ester having the structure: 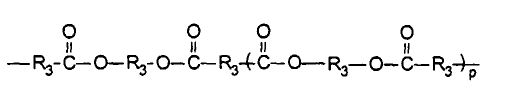
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.

3. A curable adhesive composition comprising an allylated amide compound according to claim 1 or 2, a curing initiator selected from the group consisting of a free-radical initiator and a photoinitiator, optionally a filler and optionally an adhesion promoter.

4. A curable adhesive composition comprising an allylated amide compound according to claim 1 or 2, a curing initiator selected from the group consisting of an anionic and a cationic initiator, optionally a filler, and optionally an adhesion promoter.

5. A curable adhesive composition according to claim 3 or claim 4 further comprising a compound having the structure 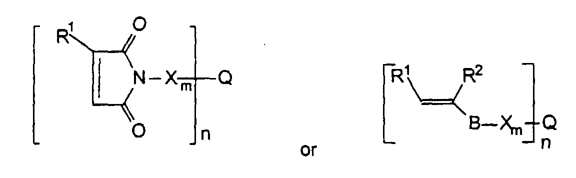 in which m is 0 or 1 and n is 1 to 6, and
(a) R¹ and R² are H or an alkyl group having 1 to 5 carbon atoms, or together form a 5 to 9 membered ring with the carbons forming the vinyl group;
(b) B is C, S, N, O, C(O), C(O)NH or C(O)N(R⁸), in which R⁸ is an alkyl group having 1 to 5 carbon atoms;
(c) X is an aromatic group selected from the group of aromatic groups having the structures: 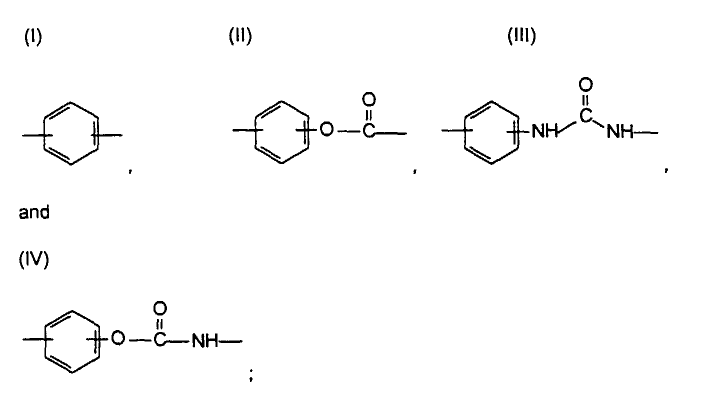 and
(d) Q is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to X; or
(d) Q is a urethane having the structure: 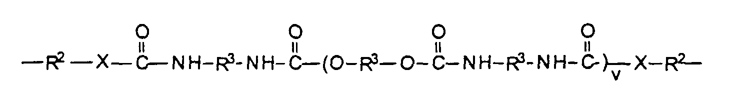
in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
(e) Q is a siloxane having the structure:
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
(f) Q is an ester having the structure: 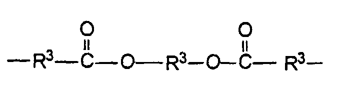
in which R³ is an alkyl or alkyloxy chain having up to 100 atoms in the chain, which chain may contain aryl substituents; or
(g) Q is an ester having the structure: 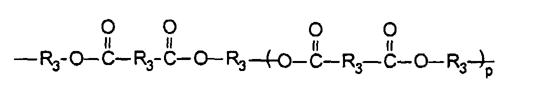
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
(h) Q is an ester having the structure: 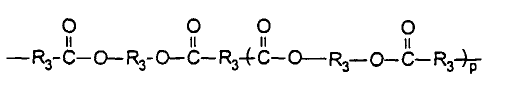
in which p is 1 to 100, and
each R³ can independently be an alkyl or alkyloxy chain having up to 100 atoms in the chain, which chain may contain aryl substituents; or
each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.

6. A curable adhesive composition according to claim 3 or claim 4 further comprising a compound having the structure 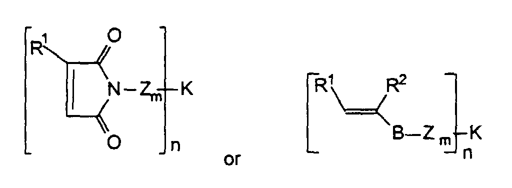 in which m is 0 or 1 and n is 1-6; and
(a) R¹ and R² are H or an alkyl group having 1 to 5 carbon atoms, or together form a 5 to 9 membered ring with the carbons forming the vinyl group;
(b) B is C, S, N, O, C(O), C(O)NH or C(O)N(R⁸), in which R⁸ is an alkyl group having 1 to 5 carbon atoms;
(c) K is an aromatic group selected from the group of aromatic groups having the structures: 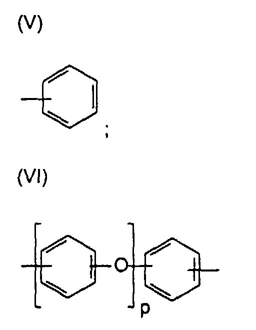 in which p is 1 to 100; 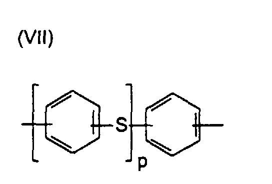 in which p is 1 to 100; 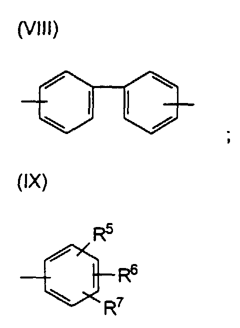 in which R⁵, R⁶, and R⁷ are a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the backbone in the chain, and in which any heteratom present may or may not be directly attached to the aromatic ring; or
R⁵, R⁶, and R⁷ are a siloxane having the structure ―(CR¹₂)ₑ―[SiR⁴₂―O]_{f}―SiR⁴₂―(CH₃)_{g}― in which the R¹ substituent is H or an alkyl group having 1 to 5 carbon atoms and the R⁴ substituent independently for each position is an alkyl group having 1 to 5 carbon atoms or an aryl group, and e is 1 to 10 and f is 1 to 50; 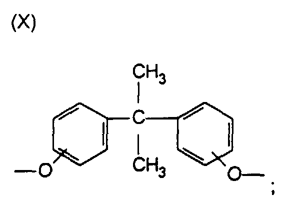 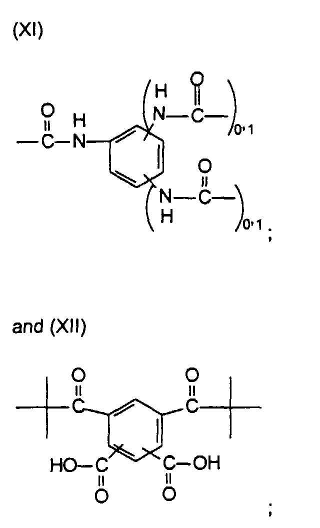
and
(d) Z is a linear or branched chain alkyl, alkyloxy, alkyl amine, alkyl sulfide, alkylene, alkyleneoxy, alkylene amine, alkylene sulfide, aryl, aryloxy, or aryl sulfide species, which may contain saturated or unsaturated cyclic or heterocyclic substituents pendant from the chain or as part of the chain, and in which any heteratom present may or may not be directly attached to K; or
(e) Z is a urethane having the structure: 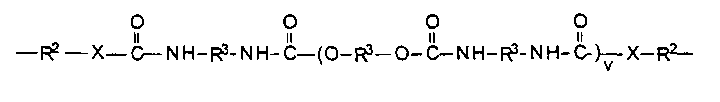
in which each R² independently is an alkyl, aryl, or arylalkyl group having 1 to 18 carbon atoms; R³ is an alkyl or alkyloxy chain having up to 100 atoms in the chain, which chain may contain aryl substituents; X is O, S, N, or P; and v is 0 to 50; or
(f) Z is a siloxane having the structure:
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, and the R⁴ substituent independently for each position is an alkyl group having 1 to 5 carbon atoms or an aryl group, and e and g are independently 1 to 10 and f is 1 to 50; or
(g) Z is an ester having the structure: 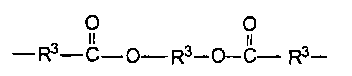
in which R³ is an alkyl or alkyloxy chain having up to 100 atoms in the chain, which chain may contain aryl substituents; or
(h) Z is an ester having the structure: 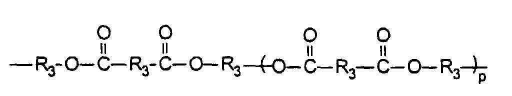
in which p is 1 to 100,
each R³ can independently be an alkyl or alkyloxy chain having up to 100 atoms in the chain, which chain may contain aryl substituents,
or a siloxane having the structure
―(CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50; or
(i) Z is an ester having the structure: 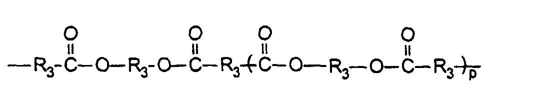
in which p is 1 to 100, and
each R³ can independently be an alkyl or alkyloxy chain having up to 100 atoms in the chain, which chain may contain aryl substituents; or
each R³ can independently be a siloxane having the structure ― (CR¹₂)ₑ―[SiR⁴₂―O]_{f}―SiR⁴₂―(CR¹₂)_{g}― in which the R¹ substituent independently for each position is H or an alkyl group having 1 to 5 carbon atoms, the R⁴ substituent independently for each position is an alkyl group having 1 to 5 carbon atoms or an aryl group, e and g are independently 1 to 10, and f is 1 to 50.

7. A method for preparing an electronic assembly comprising an electronic component adhered to a substrate comprising
(a) providing a curable adhesive according to any one of claims 3 through 10,
(b) applying the adhesive to the component or the substrate,
(c) contacting the component and the substrate, and
(d) curing the adhesive in situ.

8. An electronic assembly prepared by the method according to claim 7.
